# EUROPEAN PATENT APPLICATION

(11) **EP 4 134 374 A1**
(43) Date of publication of application: **15.02.2023**
(21) Application number: 21784541.1
(22) Date of filing: 18.03.2021
(51) Int. Cl.: C07J 71/00, C07J 5/00, A61P 19/06, A61P 35/00, A61P 35/02

(54) **COMPOUND FOR TREATING GOUT, PREPARATION METHOD THEREFOR, AND USE THEREOF**

(30) Priority: 07.04.2020 CN 202010265895
(71) Applicant: Huazhong Pharmaceutical Co., Ltd., Xiangyang, Hubei 441000 (CN)
(72) Inventor: LI, Qingsong, Beijing 100176 (CN); YAO, Linshan, Beijing 100176 (CN); GAO, Jinsheng, Beijing 100176 (CN); YUAN, Fei, Beijing 100176 (CN); CHEN, Lei, Beijing 100176 (CN); ZHANG, Hongyang, Beijing 100176 (CN)
(74) Representative: Beasley, Benjamin Oliver
(86) International application number: PCT/CN2021/081519
(87) International publication number: WO 2021/203938

(57) **Abstract**

The present invention relates to a compound for treating gout, a preparation method therefor and the use thereof. The compound is as represented by formula I. Disclosed in the present invention are a method for preparing the compound and the use of the compound in the preparation of a drug for treating or preventing gout. Further disclosed in the present invention is a composition product containing the compound. The compound provided by the present invention can be used for treating inflammation and gout, and can also be used for treating cancers.

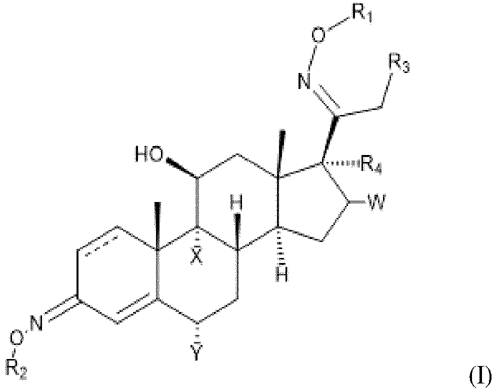

## Description

### Cross-reference to related application

The present application claims priority to Chinese Patent Application No. 202010265895.7, entitled "COMPOUND FOR TREATING GOUT, PREPARATION METHOD THEREFOR, AND USE THEREOF", and filed on April 7, 2020, the entire disclosure of which is incorporated herein by reference in its entirety.

### Technical Field

The present invention relates to a compound for treating gout, which can be used for treating gout and in particular, has a significant treatment effect on gout in the acute stage. The present invention also provides a preparation method and use of the compound.

### Background Art

Gout is a clinical syndrome caused by the deposition of sodium urate or sodium urate crystals from supersaturated extracellular fluid into tissues or organs. The deposition of urate crystals into soft tissues due to the long-term increase of blood uric acid is one of the main causes of gout. At present, western medicines for treating gout on the market can treat gout through different mechanisms, but there are problems of relatively large toxic adverse reactions and poor efficacy.

At present, clinically there are many western medicines for treating gout in the acute stage, including colchicine, non-steroidal anti-inflammatory drugs, glucocorticoids and anti-inflammatory factor drugs and the like. Colchicine may cause diarrhea, abdominal pain, liver damage, and dose-dependent adverse reactions; non-steroidal anti-inflammatory drugs including sodium salicylate, aspirin, ibuprofen, indomethacin, naproxen, and rofecoxib are prone to cardiovascular diseases; and the research and development cost of cytokine antagonists is high and the medication has limitations.

At present, glucocorticoid drugs include prednisone and methylprednisolone (moderate-acting glucocorticoids, whose glucose-raising and anti-inflammatory effects are not optimal, but the side effects are also small, and the negative feedback effect on the HPA axis, the effects on water and electrolytes are still within an acceptable range) for the treatment of gout, which are suitable for patients of gout in the acute stage who cannot tolerate non-steroidal anti-inflammatory drugs, colchicine or renal insufficiency. Methylprednisolone has a clinically fast onset effect, can rapidly inhibit the activity of enzymes and saturate hormone-specific receptors. Methylprednisolone has a fast clinical onset time, which can rapidly inhibit enzyme activity, and saturate hormone-specific receptors.

### Summary of the Invention

Described herein is a compound for treating gout. Further described herein is a method for synthesizing such compounds, a method for treating diseases including diseases in which the compound provides therapeutic benefits to patients suffering from the diseases using such compounds. Also described herein are combination products comprising the compound, including compositions, pharmaceutical formulations and kits.

### Compound

Unless otherwise specified, for the compound described herein, the following techniques may be used: conventional mass spectrometry, NMR, HPLC, protein chemistry, biochemistry, recombinant DNA techniques and pharmacological methods known to a person skilled in the art.

Compound of formula I described herein also include those of formula II.

Compound described herein includes a compound of formula I or a tautomer, a mesomer, a racemate, an enantiomer, a diastereomer, a prodrug or a mixture thereof; or pharmaceutically acceptable salts or solvates or hydrates or polymorphs of the compound of formula I or its tautomer, mesomer, racemate, enantiomer, diastereomer, prodrug or a mixture thereof: 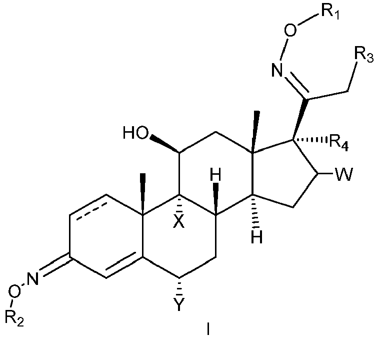 wherein,
R₁ is -H, (C₁-C₄) alkyl or -CH₂-Ar (aryl);
R₂ is -H, (C₁-C₄) alkyl or -CH₂-Ar (aryl);
R₃ is -H, -OH, halogen, -OC(=O)-(CH₂)n-CH₃ or -OC(=O)-Ar; n is an integer from 0 to 4;
R₄ is -H, -OH, -OC(=O)-(CH₂)m-CH₃ or -OC(=O)-Ar; m is an integer from 0 to 4;
X and Y are independently selected from H, -CH₃ or halogen;
W is H, -CH₃, or 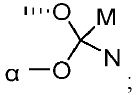 and
M and N are independently selected from H, (C₁-C₄) alkyl, -Ar (aryl), heteroaryl or benzyl.

In certain embodiments, the dotted line between positions of C-1 and C-2 indicates that it may be a double bond in addition to a single bond.

In certain embodiments, the present invention relates to any of the above compounds, wherein: R₁ is -H or -CH₂CH₃.

In certain embodiments, the present invention relates to any of the above compounds, wherein R₂ is -H or -CH₂CH₃.

In certain embodiments, the present invention relates to any of the above compounds, wherein R₃ is -H, -OH, -Cl or -OAc, 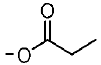 or -CH₂CH₃.

In certain embodiments, the present invention relates to any of the above compounds, wherein: R₄ is -H, -OH, or

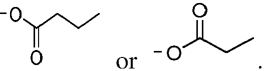

In certain embodiments, the present invention relates to any of the above compounds, wherein: X is -H, -F or -Cl.

In certain embodiments, the present invention relates to any of the above compounds, wherein: Y is -H, -CH₃, -F or -Cl.

In certain embodiments, the present invention relates to any of the above compounds, wherein: W is -H, α-CH₃, β-CH₃, α-OH, β-OH,

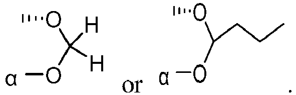

In certain embodiments, the present invention relates to any of the above compounds, wherein: R₁ is -H or -CH₂CH₃.

In certain embodiments, the present invention relates to any of the above compounds, wherein R₂ is -H or -CH₂CH₃.

In certain embodiments, the present invention relates to any of the above compounds, wherein R₃ is -OH or -OAc.

In certain embodiments, the present invention relates to any of the above compounds, wherein: R₄ is -OH or -OAc.

In certain embodiments, the present invention relates to any of the above compounds, wherein: X is -H.

In certain embodiments, the present invention relates to any of the above compounds, wherein: Y is -H or -CH₃.

In certain embodiments, the present invention relates to any of the above compounds, wherein: W is -H.

In certain embodiments, the present invention relates to any of the above compounds, wherein -C=N-O-R₂ comprises two configurations of Z/E and can be determined by hydrogen bonding and steric hindrance, and the oxime group at carbon 20 (side chain 17) in the compound I are all E configuration (trans).

In another embodiment, when W is a group of of formula II: 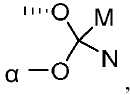 the compound is a compound 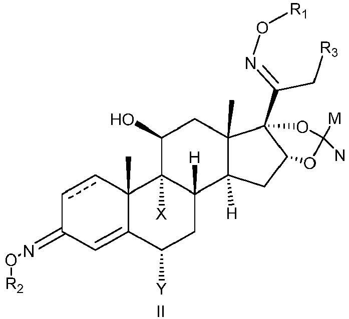 or a tautomer, a mesomer, a racemate, an enantiomer, a diastereomer, a prodrug or a mixture thereof; or pharmaceutically acceptable salts or solvates or hydrates or polymorphs of the compound of formula II or its tautomer, mesomer, racemate, enantiomer, diastereomer, prodrug or a mixture thereof, wherein R₁, R₂, R₃, X, Y, M and N have the same definitions as defined above.

In certain embodiments, in a compound of formula II or a tautomer, a mesomer, a racemate, an enantiomer, a diastereomer, a prodrug or a mixture thereof; or pharmaceutically acceptable salts or solvates or hydrates or polymorphs of the compound of formula II or its tautomer, mesomer, racemate, enantiomer, diastereomer, prodrug or a mixture thereof of the present invention, M and N are independently selected from H, (C₁-C₄) alkyl group, -Ar, heteroaryl or benzyl.

In certain embodiments, the present invention relates to compounds numbered 1-17 in the table below or their tautomers, mesomer, racemates, enantiomers, diastereomers, prodrugs or mixture thereof; or pharmaceutically acceptable salts or solvates or hydrates or polymorphs of the compounds or their tautomers, mesomer, racemates, enantiomers, diastereomers, prodrugs or mixtures thereof:

| No. | (Z/E) configuration | R₁ | R₂ | R₃ | R₄ | C-1~2 | X | Y | W |
|---|---|---|---|---|---|---|---|---|---|
| 1 | 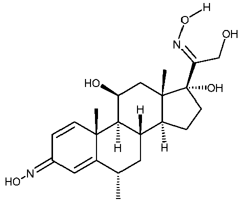 | -H | -H | -OH | -OH | 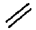 | -H | -CH₃ | -H |
| 2 | 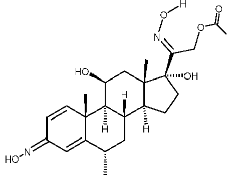 | -H | -H | -OAc | -OH | 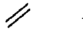 | -H | -CH₃ | -H |
| 3 | 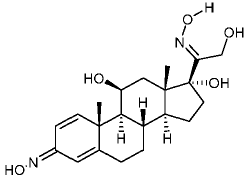 | -H | -H | OH | -OH | 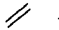 | -H | -H | -H |
| 4 | 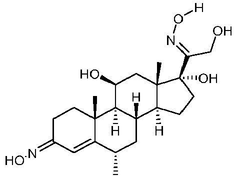 | -H | -H | -OH | -OH | 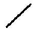 | -H | -CH₃ | -H |
| 5 | 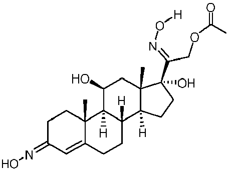 | -H | -H | -OAc | -OH | 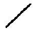 | -H | -H | -H |
| 6 | 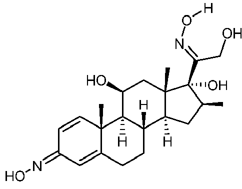 | -H | -H | -OH | -OH | 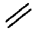 | -H | -H | β-CH₃ |
| 7 | 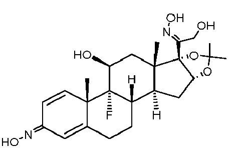 | -H | -H | -OH | -- | 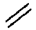 | -F | -H | 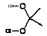 |
| 8 | 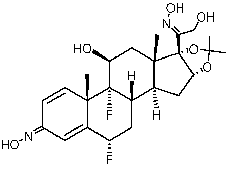 | -H | -H | -OH | -- | 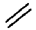 | -F | -F | 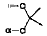 |
| 9 | 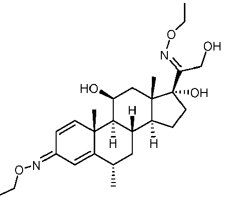 | -CH₂CH₃ | -CH₂CH₃ | -OH | -OH | 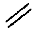 | -H | -CH₃ | -H |
| 10 | 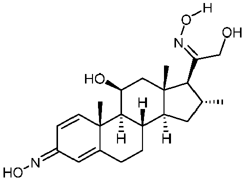 | -H | -H | -OH | -H | 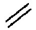 | -H | -H | α-CH₃ |
| 11 | 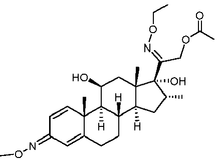 | -CH₂CH₃ | -CH₂CH₃ | -OAc | -OH | 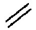 | -H | -H | α-CH₃ |
| 12 | 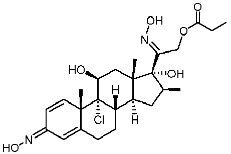 | -H | -H | -OAc | -OH | 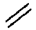 | -Cl | -H | β-CH₃ |
| 13 | 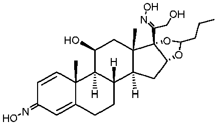 | -H | -H | -OH | -- | 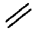 | -H | -H | 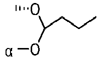 |
| 14 | 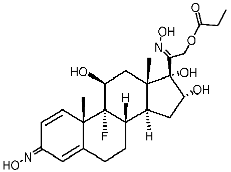 | -H | -H | 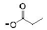 | -OH | 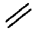 | -F | -H | α-OH |
| 15 | 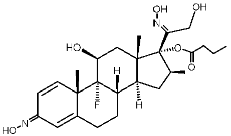 | -H | -H | -OH | 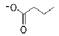 | 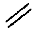 | -F | -H | β-CH₃ |
| 16 | 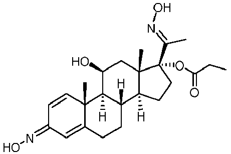 | -H | -H | -H | 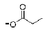 | 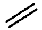 | -H | -H | -H |
| 17 | 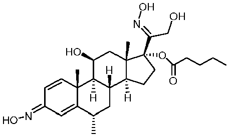 | -H | -H | -OH | 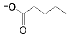 | 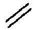 | -H | -CH₃ | -H |

Many disorders may be treated with the compounds described herein. For example, the compounds described herein can be useful for the treatment of gout, particularly with significant therapeutic effects for gout in the acute stage. On the other hand, the substituents in the compounds described herein can prolong the metabolic cycle of the compounds *in vivo,* and have relatively more moderate and long-acting anti-gout effects.

The compounds described herein also have strong anti-inflammatory effect, mainly on regulating the expression of glucocorticoid receptor a (Gra), glucocorticoid receptor b (GRb), proinflammatory factor TNF-α and inflammatory protective factor IL-10 in the inflammatory response pathway. The compounds described herein are therefore also useful in the treatment of inflammation.

The compounds described herein are also useful in the treatment of cancers (or tumors), such as human lung adenocarcinoma, human acute lymphoblastic leukemia, human B cell lymphoma, human breast carcinoma, human malignant glioblastoma, human live cancer, human cervical cancer, human non-small cell lung cancer, human osteosarcoma, human colon cancer, human osteoblastic sarcoma, human Burkitt's lymphoma, and various tumors.

### Preparation Method

### General synthetic method

In some embodiments, the compounds of the present invention may be prepared using the methods described in synthetic routes I-IV below.

In synthetic route I, under the alkaline condition, the carbonyl group in the substrate is reacted with hydroxylamine hydrochloride, and the oxime group at the R₁ and/or R₂ positions in the general formula can be easily prepared.

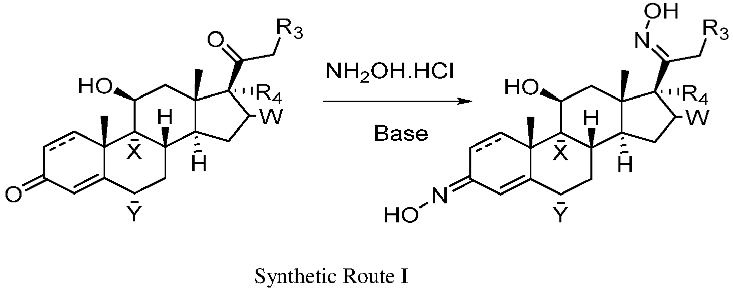

In synthetic route II, under the alkaline condition, hydroxyl group in the substrate is reacted with an acylation reagent (such as ROCl, specifically, acid chloride), and the acylation products corresponding to the R₃ and/or R₄ positions in the general formula can be easily prepared. In the preparation of the compounds of Examples 2, 5, 11, 14, 15, 16, 17 of the present invention, synthetic route II can be used, wherein R is a linear saturated alkyl, such as a linear saturated (C₁-C₁₂) alkyl, especially a linear saturated (C₁-C₄) alkyl, and specific examples include methyl, ethyl, n-propyl or n-butyl.

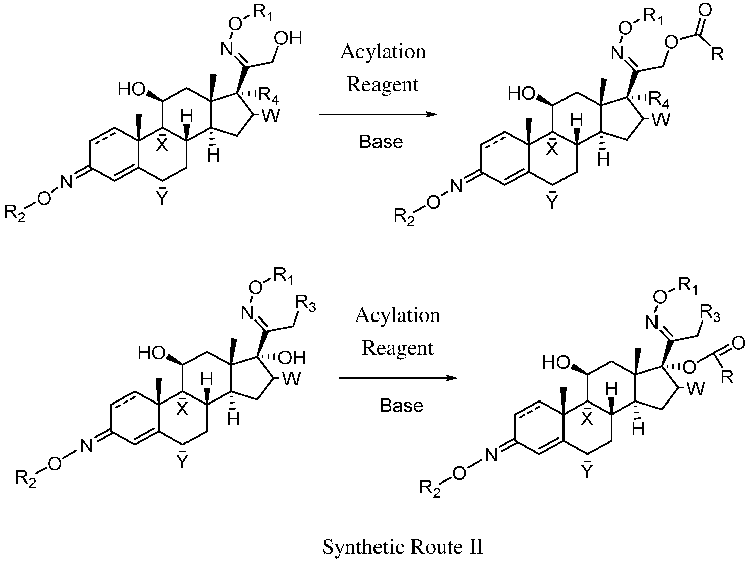

In synthetic route III, under the acidic condition, the ortho-dihydroxy group in the R₄ and W positions in the substrate is reacted with ketones (such as acetone) or aldehydes (such as butyraldehyde) to form ketals/acetals. In the preparation of the compounds of Examples 7, 8 and 13 of the present invention, the procedure in synthetic scheme III can be used. In the preparation of the compound of Example 14, the ortho-dihydroxy groups at the R₄ and W positions in the substrate are first protected with ketals, and then reacted with an acylation reagent (such as ROCl, specifically, acid chloride), and the ketals are removed under acidic conditions to obtain the product, wherein R is a linear saturated alkyl, for example a linear saturated (C₁-C₁₂) alkyl, in particular a linear saturate (C₁-C₄) alkyl, specific examples including methyl, ethyl, n-propyl or n-butyl.

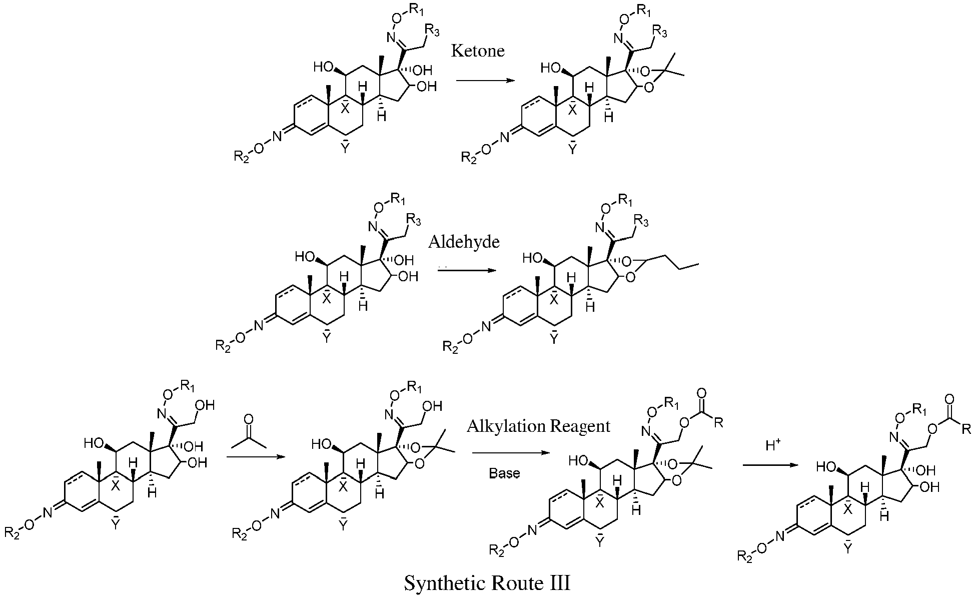

In synthetic route IV, under the alkaline condition, the oxime group in the substrate is reacted with an acylation reagent (e.g., iodoethane) to form an alkyl oxime. In the preparation of the compounds of Examples 9 and 11 of the present invention, synthetic route IV can be used.

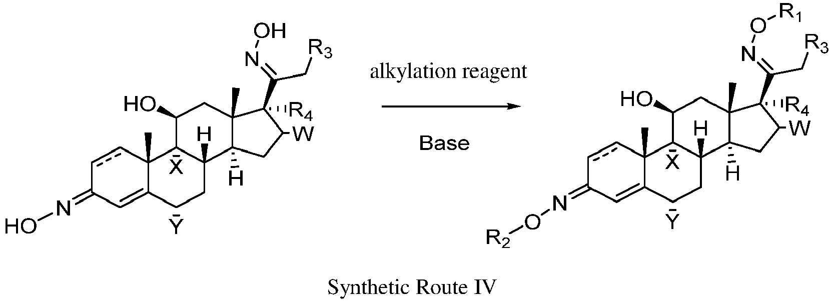

In the preparation methods above, R₁, R₂, R₃, R₄, X, Y, W, M and N have the same definitions as above.

The above preparation methods may also include separation and purification of the product, which may be carried out using method commonly employed in organic synthesis, such as filtration, extraction, washing, concentration, chromatography and the like in a suitable combination.

### Use

The present invention also provides use of the compound or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof described above in the preparation of a drug for treating or preventing gout, or in the preparation of a drug for treating or preventing inflammation; or in the preparation of a drug for treating or preventing cancer (or tumor).

Preferably, the drug further comprises a pharmaceutically acceptable auxiliary material such as a diluent, an excipient and/or a binder.

Wherein the gout is preferably a gout in the acute stage.

In particular, the inflammation is an inflammation associated with gout in the acute stage.

The inflammation is preferably an inflammation associated with regulating any one or more of the expressions of glucocorticoid receptor GRa, glucocorticoid receptor GRb, proinflammatory factor TNF-α, and inflammatory protective factor IL-10 in the inflammatory response pathway.

The cancer is selected from any one or more of human lung adenocarcinoma, human acute lymphoblastic leukemia, human B cell lymphoma, human breast carcinoma, human malignant glioblastoma, human live cancer, human cervical cancer, human non-small cell lung cancer, human osteosarcoma, human colon cancer, human osteoblastic sarcoma, and human Burkitt's lymphoma.

Further, the compound or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof in the use is as the sole active ingredient or in combination with other biologically active substances.

The other biologically active substances include colchicine, non-steroidal anti-inflammatory drugs (including sodium salicylate, aspirin, ibuprofen, indomethacin, naproxen and rofecoxib), glucocorticoids (including prednisone and methylprednisolone), anti-inflammatory drugs, methylprednisolone and the like.

### Combination product

The present invention also provides a combination product for treating or preventing gout or inflammation or cancer (or tumor), comprising one or more of the above compounds or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof.

Preferably, the combination product further comprises a pharmaceutically acceptable auxiliary material such as a diluent, excipient and/or a binder.

In certain embodiments, the combination product is also referred to as a pharmaceutical composition.

Preferably, the combination product is a kit.

### Treating Method

The present invention also provides a method for treating or preventing gout or inflammation or cancer, comprising: administering to a subject or patient in need thereof, orally or non-orally, an effective amount of the above compound or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof, or the above combination product.

Wherein the gout is preferably a gout in the acute stage.

The inflammation may or may not be associated with gout.

Preferably, the inflammation is an inflammation associated with gout in the acute stage.

The inflammation is preferably an inflammation associated with regulating any one or more of the expressions of glucocorticoid receptor GRa, glucocorticoid receptor GRb, proinflammatory factor TNF-α, and inflammatory protective factor IL-10 in the inflammatory response pathway.

The cancer is selected from any one or more of human lung adenocarcinoma, human acute lymphoblastic leukemia, human B cell lymphoma, human breast carcinoma, human malignant glioblastoma, human live cancer, human cervical cancer, human non-small cell lung cancer, human osteosarcoma, human colon cancer, human osteoblastic sarcoma, and human Burkitt's lymphoma.

The oral or non-oral route can be delivery to the subject or patient by one or more of oral administration, injection, patch, spray, and other known routes. The effective amount can include an amount effective to treat, reduce, relieve, alleviate or eliminate one or more symptoms of a condition sought to be treated or alternatively sought to be avoided, or an amount effective to additionally generate clinically identifiable advantageous changes in the condition or its effect.

In certain embodiments, the compounds provided herein are administered to a mammal.

In certain embodiments, the compounds provided herein are administered to a human.

In certain embodiments, the compounds provided herein are administered orally.

In other embodiments, the compounds provided herein are used in the formulation of drugs for inhibiting and/or treating gout. In some other embodiments, the compounds provided herein are used in the formulation of drugs for inhibiting and/or treating gout in the acute stage.

### Definition of Terms

Alkyl refers to a linear or branched chain saturated hydrocarbyl substituent (i.e., a substituent obtained by removing hydrogen from a hydrocarbon) containing 1 to 12 carbon atoms. Examples of such substituents include methyl, ethyl, propyl (including n-propyl and isopropyl), butyl (including n-butyl, isobutyl, sec-butyl and tert-butyl), pentyl, isopentyl, hexyl and the like.

Aryl (-Ar), refers to an aromatic substituent comprising one ring or two or three fused rings. The aryl group may have 6 to 18 carbon atoms. As an example, the aryl may have 6 to 14 carbon atoms. The term "aryl" may refer to a substituent such as phenyl, naphthyl and anthryl.

Halogen, refers to fluorine (which may be represented as F), chlorine (which may be represented as Cl), bromine (which may be represented as Br), or iodine (which may be represented as I). In one embodiment, the halogen is chlorine. In another embodiment, the halogen is fluorine. In another embodiment, the halogen is bromine.

The term "formula I and formula II" as used herein may be referred to as "compound of the present invention". Such terms are also defined to include all forms of compounds of formula I and formula II, including hydrates, solvates, isomers, crystalline and amorphous forms, isomorphs, polymorphs and metabolites thereof. For example, the compounds of formula I and formula II and pharmaceutically acceptable salt thereof may be present in both unsolvated and solvated form. When the solvent or water is tightly bound, the complex will have a well-defined stoichiometry independent of humidity. However, when the solvent or water is weakly bound, such as in channel solvate and hygroscopic compound, the water/solvent content will depend on the humidity and drying conditions. In such cases, non-stoichiometry would become the norm.

The compound of the present invention may have asymmetric carbon atoms. The C-C bond of the compound of the present invention may be shown herein using solid line, solid wedge, or dashed wedge. The use of solid line to illustrate bond to asymmetric carbon atom is intended to mean that all possible stereoisomers (e. g., specific enantiomer, racemic mixtures, etc.) of the carbon atom are included. The use of solid or dashed wedges to show bonds to asymmetric carbon atom is intended to mean that only the stereoisomers shown are intended to be included. It is possible that the compound of the present invention may contain more than one asymmetric carbon atom. In these compounds, the bonds to asymmetric carbon atom shown using solid lines are intended to mean that all possible stereoisomers are intended to be included. For example, unless otherwise indicated, the compounds of the present invention are intended to be present as enantiomers and diastereomers or as racemates and mixtures thereof. The use of solid lines to show bonds to one or more asymmetric carbon atoms in the compound of the present invention and the use of solid or dashed wedges to show bonds to other asymmetric carbon atoms in the same compound are intended to indicate the presence of a mixture of diastereomers.

Stereoisomers of the compounds of the present invention include cis and trans isomers, optical isomers such as R and S enantiomers, diastereomers, geometric isomers, rotamers, conformers, and tautomers of the compounds of the present invention, including compounds that exhibit more than one type of isomerisms; and mixture thereof (e.g., racemates and diastereomers). Also included are acid addition salts or base addition salts in which the counterions are optically active, for example, D-lactate or L-lysine, or racemic, for example, DL-tartrate or DL-quinone amino acid.

When any of the racemate crystallizes, there may be two different types of crystals. The first type is the racemic compound (true racemate) mentioned above, in which a crystal is produced which contains an equimolar amount of the two enantiomers in a homogeneous form. The second type is a racemic mixture or aggregate in which equimolar amounts of the two forms of crystals are produced, each containing a single enantiomer.

Among them, -C=N-O-R₂ comprises two configurations of Z/E and can be determined by hydrogen bonding and steric hindrance, and the oxime group at carbon 20 (side chain 17) in compound I are all E configuration (trans).

Generally, the compounds of the present invention are administered in an amount effective to treat the conditions described herein. The compounds of the present invention are administered by any suitable route in the form of a pharmaceutical composition suitable for such a route and at a dose effective for the intended treatment. The therapeutically effective amount of a compound required to treat the progression of a medical condition is readily determined by a person skilled in the art using preclinical and clinical methods familiar in the field of medicine. As used herein, the term "therapeutically effective amount" refers to the amount of a compound administered that provides some relief from one or more symptoms of the condition being treated.

Unless otherwise indicated, the practice of the present invention uses conventional organic chemistry, pharmacology, molecular biology (including recombinant technology), cell biology, biochemistry, and immunology techniques. Such techniques are described in the existing literature, each of which is incorporated herein by reference in its entirety.

### Specific Modes for Carrying Out the Embodiments

The present invention is described in further detail below in conjunction with specific Examples, but is not intended to limit the scope of the present invention.

The raw material can all available from open commercial sources unless otherwise specified.

### Example 1

### (6S, 8S, 9S, 10R, 11S, 13S, 14S, 17R)-11,17-dihydroxy-17-((E)-2-hydroxy-1-(hydroxyimino)ethyl)-6,10,13-trimethyl-6,7,8,9,10,11,12,13,14,15,16,17-dodecahydro-3H-cyclopentane[a]phenanthrene-3-one oxime

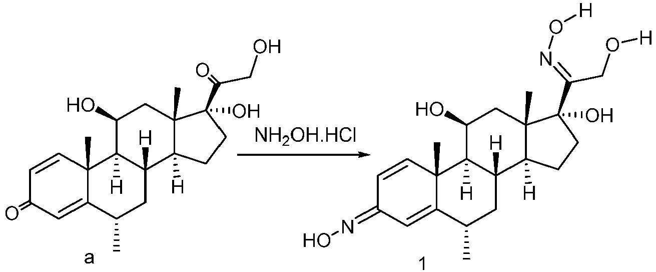

To a solution of methylprednisolone a (500 mg, 1.0 eq) in methanol at room temperature was added sodium acetate (370 mg, 2.5 eq), and hydroxylamine hydrochloride (203 mg, 2.2 eq) was added in batches, to react at room temperature for 6 h. After TLC monitoring the reaction was completed, the solvent was spun dry in vacuo. The resultant was purified under EA and PE gradients using a Biotage Isolera automated column chromatograph, and then dried under reduced pressure. Z/E-1 (510 mg, 95%) was obtained as an off-white solid. The resultant was then further purified by RPHPLC under a gradient of MeOH and H₂O, and dried under reduced pressure to give pure compound 3(Z)-1 (200 mg) and compound 3(E)-1 (248 mg).

Compound 3(Z)-1: ¹H-NMR (600MHz, d₆-DMSO) δ_{H} 0.629(m, 1H), 0.870-0.835 (m, 4H), 1.051-1.035 (d, 3H), 1.254-1.119 (m,1H), 1.323(s, 3H), 1.436-1.354 (m, 1H), 1.604-1.590 (m, 2H), 1.758-1.714(m, 2H), 1.995-1.969(m, 2H), 2.636-2.536(m, 1H), 2.734(m, 1H), 4.164-4.132(m, 1H), 4.274-4.225(m, 2H), 4.406-4.397(m, 1H), 4.589-4.560(m, 1H), 4.807(s, 1H), 6.116-6.087(m, 1H), 6.354-6.329(d, 1H), 6.446(s, 1H), 10.576(s, 1H), 10.796(s, 1H); ¹³C(600MHz, d₆-DMSO) δ_{C} 18.24, 18.62, 23.45, 23.60, 31.71, 32.73, 32.83, 39.96, 43.51, 43.67, 47.07, 50.90, 55.74, 56.94, 67.76, 85.67, 104.83, 121.59, 141.73, 148.76, 159.72, 161.47 ; Calculated value 405.3, Experimental value 405.2 for LRMS(ES+)[ C₂₂H₃₂N₂O₅+H].

Compound 3(E)-1: ¹H-NMR (600MHz, d₆-DMSO) δ_{H} 0.632-0.612(m, 1H), 0.875-0.832 (m, 4H), 1.032-1.021 (d, 3H), 1.254-1.119 (m,1H), 1.324(s, 3H), 1.368-1.354 (m, 1H), 1.607-1.595 (m, 2H), 1.760-1.709(m, 2H), 2.004-1.943(m, 2H), 2.511(m, 1H), 2.731(m, 1H), 4.157-4.127(m, 1H), 4.266-4.233(m, 2H), 4.408-4.402(m, 1H), 4.592-4.572(m, 1H), 4.809(s, 1H), 5.708(s, 1H), 6.519-6.501(d, 1H), 6.779-6.759(m, 1H), 10.522(s, 1H), 10.793(s, 1H); ¹³C(600MHz, d₆-DMSO) δ_{C} 18.23, 18.57, 23.31, 23.48, 31.74, 32.64, 32.83, 39.95, 43.42, 43.62, 47.03, 50.92, 55.76, 56.69, 67.70, 85.71, 112.20, 113.75, 145.96, 149.06, 156.86, 159.72 ; Calculated value 405.3, Experimental value 405.2 for LRMS(ES+)[ C₂₂H₃₂N₂O₅+H].

### Example 2

### (2E)-2-((6S,8S,9S,10R,11S,13S,14S,17R)-11,17-dihydroxy-3-(hydroxyimino)-6,10,13-trimethyl-6,7,8,9,10,11,12,13,14,15,16,17-dodecahydro-3H-cyclopentane[a]phenanthrene-17-yl)-2-(hydroxyimino) ethyl acetate

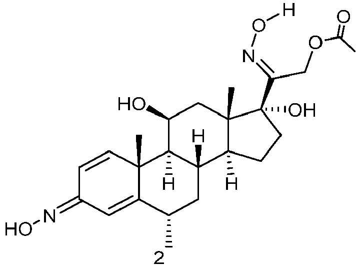

Compound 2 was prepared according to the methods shown in Synthesis Routes I and II in the general synthetic methods above using appropriate starting materials and separated to give 3(Z)-2 and 3(E)-2.

Compound 3(Z)-2: ¹H-NMR (600MHz, d₆-DMSO) δ_{H} 0.630(m, 1H), 0.863-0.835 (m, 4H), 1.044-1.032 (d, 3H), 1.255-1.122 (m,1H), 1.325(s, 3H), 1.366-1.352 (m, 1H), 1.603-1.590 (m, 2H), 1.758-1.712(m, 2H), 1.998-1.953(m, 2H), 2.632(m, 1H), 2.688(s, 3H), 2.733(m, 1H), 4.166-4.142(m, 1H), 4.259-4.227(m, 2H), 4.405-4.386(m, 1H), 4.806(s, 1H), 6.113-6.078(m, 1H), 6.345-6.327(d, 1H), 6.452(s, 1H), 10.574(s, 1H), 10.793(s, 1H); ¹³C(600MHz, d₆-DMSO) δ_{C} 18.22, 18.56, 22.93, 23.39, 23.60, 31.72, 32.69, 32.81, 39.94, 43.52, 43.66, 47.07, 50.92, 55.75, 56.92, 67.74, 85.65, 104.81, 121.60, 141.71, 148.78, 159.73, 161.47, 168.91 ; Calculated value 447.3 Experimental value 447.2 for LRMS(ES+)[ C₂₄H₃₄N₂O₆+H].

Compound 3(E)-2: ¹H-NMR (600MHz, d₆-DMSO) δ_{H} 0.628(m, 1H), 0.865-0.840 (m, 4H), 1.038-1.025 (d, 3H), 1.252-1.120 (m,1H), 1.327(s, 3H), 1.368-1.353 (m, 1H), 1.605-1.596 (m, 2H), 1.761-1.718(m, 2H), 2.002-1.956(m, 2H), 2.598(m, 1H), 2.730(m, 4H), 4.157-4.127(m, 1H), 4.263-4.237(m, 2H), 4.404-4.400(m, 1H), 4.807(s, 1H), 5.705(s, 1H), 6.518-6.502(d, 1H), 6.781-6.758(m, 1H), 10.524(s, 1H), 10.781(s, 1H); ¹³C(600MHz, d₆-DMSO) δ_{C} 18.25, 18.55, 22.88, 23.35, 23.49, 31.72, 32.63, 32.82, 39.95, 43.54, 43.61, 47.04, 50.92, 55.75, 56.70, 67.72, 85.70, 112.22, 113.74, 145.93, 149.07, 156.86, 159.73, 168.95 ; Calculated value 447.3 Experimental value 447.2 for LRMS(ES+)[ C₂₄H₃₄N₂O₆+H].

### Example 3

### (8S,9S,10R,11S,13S,14S,17R)-11,17-dihydroxy-17-((E)-2-hydroxy-1-(hydroxyimino)ethyl)-10,13-dimethyl-6,7,8,9,10,11,12,13,14,15,16,17-dodecahydro-3H-cyclopentane[a]phenanthrene-3-one oxime

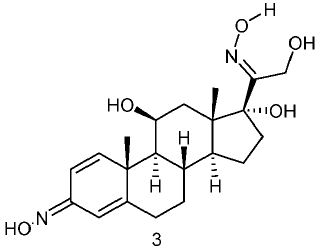

Compound 3 was prepared according to the method shown in Synthesis Route I in the general synthetic methods above using appropriate starting materials and separated to give (3*Z*)-3 and (3*E*)-3.

Compound 3(Z)-3: ¹H-NMR (600MHz, d₆-DMSO) δ_{H} 0.630(m, 1H), 0.869-0.834 (m, 4H), 1.253-1.120 (m,1H), 1.325(s, 3H), 1.368-1.352 (m, 1H), 1.601-1.592 (m, 2H), 1.756-1.713(m, 2H), 1.998-1.971(m, 2H), 2.602-2.523(m, 2H), 2.735(m, 1H), 4.162-4.131(m, 1H), 4.273-4.224(m, 2H), 4.402-4.394(m, 1H), 4.591-4.557(m, 1H), 4.806(s, 1H), 6.114-6.085(m, 1H), 6.352-6.327(d, 1H), 6.448(s, 1H), 10.573(s, 1H), 10.798(s, 1H); ¹³C(600MHz, d₆-DMSO) δ_{C} 18.23, 23.47, 23.58, 31.73, 32.49, 32.84, 39.93, 43.58,43.67, 47.06, 50.92, 55.75, 56.94, 67.77, 85.65, 104.82, 121.60, 141.72, 148.76, 159.73, 161.49 ; Calculated value 391.3 Experimental value 391.2 for LRMS(ES+)[ C₂₁H₃₀N₂O₅+H].

Compound 3(E)-3: ¹H-NMR (600MHz, d₆-DMSO) δ_{H} 0.627(m, 1H), 0.872-0.835 (m, 4H), 1.250-1.122 (m,1H), 1.323(s, 3H), 1.364-1.357 (m, 1H), 1.606-1.598 (m, 2H), 1.768-1.709(m, 2H), 2.001-1.939(m, 2H), 2.589-2.478(m, 2H), 2.732(m, 1H), 4.153-4.125(m, 1H), 4.263-4.231(m, 2H), 4.409-4.404(m, 1H), 4.590-4.574(m, 1H), 4.810(s, 1H), 5.707(s, 1H), 6.518-6.502(d, 1H), 6.779-6.762(m, 1H), 10.524(s, 1H), 10.796(s, 1H); ¹³C(600MHz, d₆-DMSO) δ_{C} 18.24, 23.34, 23.47, 31.75, 32.47, 32.86, 39.94, 43.53, 43.64, 47.02, 50.94, 55.73, 56.69, 67.68, 85.72, 112.23, 113.74, 145.95, 149.06, 156.83, 159.74 ; Calculated value 391.3 Experimental value 391.2 for LRMS(ES+)[ C₂₁H₃₀N₂O₅+H].

### Example 4

### (6S,8S,9S,10R,11S,13S,14S,17R)-11,17-dihydroxy-17-((E)-2-hydroxy-1-(hydroxyimino)ethyl)-6,10,13-trimethyl-1,2,6,7,8,9,10,11,12,13,14,15,16,17-tetrahydro-3H-cyclopentane[a]phenanthrene-3-one oxime

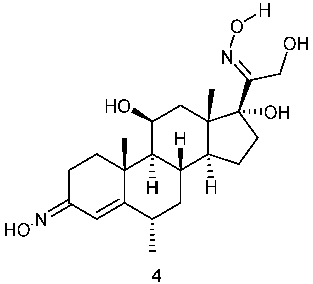

Compound 4 was prepared according to the methods shown in Synthesis Route I in the general synthetic methods above using appropriate starting materials and separated to give (3Z)-4 and (3E)-4.

Compound 3(Z)-4: ¹H-NMR (600MHz, d₆-DMSO) δ_{H} 0.635(m, 1H), 0.868-0.837 (m, 4H), 1.053-1.032 (d, 3H), 1.085-1.089(m, 1H), 1.156-1.127 (m, 1H), 1.284-1.277(m, 1H), 1.323(s, 3H), 1.371-1.352 (m, 1H), 1.603-1.585 (m, 2H), 1.759-1.713(m, 2H), 1.996-1.965(m, 2H), 2.636-2.534(m, 1H), 2.738(m, 1H), 2.979-2.965(m, 2H), 4.166-4.135(m, 1H), 4.273-4.226(m, 2H), 4.405-4.399(m, 1H),

4.588-4.561(m, 1H), 4.809(s, 1H), 6.407(s, 1H), 10.127(s, 1H), 10.791(s, 1H); ¹³C(600MHz, d₆-DMSO) δ_{C} 18.22, 18.65, 23.47, 23.61, 30.82, 31.71, 32.74, 32.84, 38.75, 39.95, 43.52, 43.68, 47.05, 50.92, 55.73, 56.96, 67.73, 85.65, 101.52, 148.59, 159.71, 162.47 ; Calculated value 407.3 Experimental value 407.2 for LRMS(ES+)[ C₂₂H₃₂N₂O₅+H].

Compound 3(E)-4: ¹H-NMR (600MHz, d₆-DMSO) δ_{H} 0.623(m, 1H), 0.873-0.834 (m, 4H), 1.035-1.026 (d, 3H), 1.089-1.082(m, 1H), 1.156-1.117 (m,1H), 1.282-1.274(m, 1H), 1.325(s, 3H), 1.367-1.358 (m, 1H), 1.609-1.597 (m, 2H), 1.759-1.708(m, 2H), 2.001-1.945(m, 2H), 2.514(m, 1H), 2.733(m, 1H), 3.124-3.037(m, 2H), 4.154-4.123(m, 1H), 4.268-4.232(m, 2H), 4.406-4.401(m, 1H), 4.590-4.575(m, 1H), 4.811(s, 1H), 5.701(s, 1H), 10.124(s, 1H), 10.791(s, 1H); ¹³C(600MHz, d₆-DMSO) δ_{C} 18.23, 18.56, 23.33, 23.47, 30.21, 31.78, 32.02, 32.84, 39.07, 39.93, 43.44, 43.65, 47.01, 50.93, 55.74, 56.69, 67.70, 85.71, 113.21, 149.03, 157.86, 159.74 ; Calculated value 407.3 Experimental value 407.2 for LRMS(ES+)[ C₂₂H₃₂N₂O₅+H].

### Example 5

### (2E)-2-((8S,9S,10R,11S,13S,14S,17R)-11,17-dihydroxy-3-(hydroxyimino)-10,13-dimethyl-2,3,6,7,8,9,10,11,12,13,14,15,16,17-tetrahydro-1H-cyclopentane[a]phenanthro-17-yl)-2-(hydroxyimino) ethyl acetate

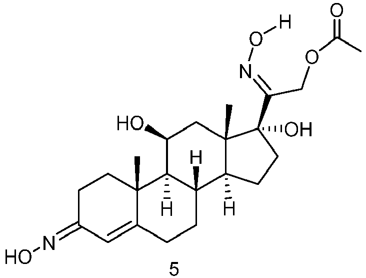

Compound 5 was prepared according to the methods shown in Synthesis Routes I and II in the general synthetic methods above using appropriate starting materials and separated to give (3Z)-5 and (3E)-5.

Compound 3(Z)-5: ¹H-NMR (600MHz, d₆-DMSO) δ_{H} 0.631(m, 1H), 0.868-0.837 (m, 4H), 1.085-1.089(m, 1H), 1.156-1.127 (m, 1H), 1.284-1.277(m, 1H), 1.323(s, 3H), 1.371-1.352 (m, 1H), 1.603-1.585 (m, 2H), 1.759-1.713(m, 2H), 1.996-1.965(m, 2H), 2.602-2.523(m, 2H), 2.688(s, 3H), 2.738(m, 1H), 2.979-2.965(m, 2H), 4.166-4.135(m, 1H), 4.273-4.226(m, 2H), 4.405-4.399(m, 1H), 4.809(s, 1H), 6.407(s, 1H), 10.127(s, 1H), 10.791(s, 1H); ¹³C(600MHz, d₆-DMSO) δ_{C} 18.22, 22.93, 23.47, 23.61, 30.82, 31.71, 32.48, 32.84, 38.75, 39.95, 43.52, 43.68, 47.05, 50.92, 55.73, 56.96, 67.73, 85.65, 101.52, 148.59, 159.71, 162.47, 168.91 ; Calculated value 435.3 Experimental value 435.2 for LRMS(ES+)[ C₂₃H₃₄N₂O₆+H].

Compound 3(E)-5: ¹H-NMR (600MHz, d₆-DMSO) δ_{H} 0.624(m, 1H), 0.872-0.835 (m, 4H), 1.091-1.078(m, 1H), 1.154-1.116 (m,1H), 1.283-1.275(m, 1H), 1.324(s, 3H), 1.366-1.359 (m, 1H), 1.610-1.595 (m, 2H), 1.754-1.706(m, 2H), 2.002-1.945(m, 2H), 2.603-2.522(m, 2H), 2.687(s, 3H), 2.735(m, 1H), 3.126-3.034(m, 2H), 4.155-4.124(m, 1H), 4.267-4.231(m, 2H), 4.408-4.403(m, 1H), 4.813(s, 1H), 5.704(s, 1H), 10.127(s, 1H), 10.793(s, 1H); ¹³C(600MHz, d₆-DMSO) δ_{C} 18.23, 22.88, 23.33, 23.47, 30.21, 31.78, 32.02, 32.44, 39.06, 39.94, 43.45, 43.66, 47.02, 50.94, 55.73, 56.68, 67.71, 85.72, 113.23, 149.07, 157.88, 159.75, 168.95 ; Calculated value 435.3 Experimental value 435.2 for LRMS(ES+)[ C₂₃H₃₄N₂O₆+H].

### Example 6

### (8S,9S,10R,11S,13S,14S,16S,17R)-11,17-dihydroxy-17-((E)-2-hydroxy-1-(hydroxyimino)ethyl)-10,13,16-trimethyl-6,7, 8,9,10,11,12,13,14,15,16,17-dodecahydro-3H-cyclopentane[a]phenanthrene-3-one oxime

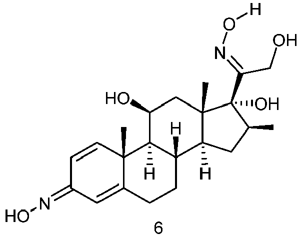

Compound 6 was prepared according to the methods shown in Synthesis Routes I and II in the general synthetic methods above using appropriate starting materials and separated to give (3*Z*)-6 and (3*E*)-6.

Compound 3(*Z*)-6: ¹H-NMR (600MHz, d₆-DMSO) δ_{H} 0.630(m, 1H), 0.870-0.832 (m, 7H), 1.252-1.123 (m,1H), 1.324(s, 3H), 1.602-1.593 (m, 2H), 1.755-1.716(m, 3H), 2.001 - 1.974(m, 2H), 2.605-2.526(m, 2H), 4.162-4.135(m, 1H), 4.272-4.228(m, 2H), 4.403-4.397(m, 1H), 4.587-4.561(m, 1H), 4.806(s, 1H), 6.112-6.084(m, 1H), 6.353-6.328(d, 1H), 6.451(s, 1H), 10.569(s, 1H), 10.793(s, 1H); ¹³C(600MHz, d₆-DMSO) δ_{C} 17.28, 18.22, 23.57, 27.38, 31.74, 32.45, 38.89, 39.93, 43.66, 43.57, 47.07, 50.90, 55.74, 56.94, 67.76, 88.93, 104.84, 121.63, 141.70, 148.75, 159.70, 161.39 ; Calculated value 405.43 Experimental value 405.3 for LRMS(ES+)[ C₂₂H₃₂N₂O₅+H].

Compound 3(E)-6: ¹H-NMR (600MHz, d₆-DMSO) δ_{H} 0.629(m, 1H), 0.872-0.8345 (m, 7H), 1.251-1.123 (m,1H), 1.325(s, 3H), 1.604-1.596 (m, 2H), 1.765-1.711(m, 3H), 2.001-1.969(m, 2H), 2.593-2.481(m, 2H), 4.152-4.137(m, 1H), 4.269-4.233(m, 2H), 4.405-4.402(m, 1H), 4.590-4.574(m, 1H), 4.810(s, 1H), 5.702(s, 1H), 6.513-6.501(d, 1H), 6.776-6.761(m, 1H), 10.523(s, 1H), 10.794(s, 1H); ¹³C(600MHz, d₆-DMSO) δ_{C} 17.27, 18.23, 23.34, 27.38, 31.76, 32.47, 38.85, 39.94, 43.56, 43.64, 47.03, 50.93, 55.74, 56.67, 67.69, 88.98, 112.24, 113.72, 145.95, 149.07, 156.85, 159.78 ; Calculated value 405.43 Experimental value 405.3 for LRMS(ES+)[ C₂₂H₃₂N₂O₅+H].

### Example 7

### (6aS,6bR,7S,8aS,8bR,11aR,12aS,12bS)-6b-fluoro-7-hydroxy-8b-((E)-2-hydroxy-1-(hydroxyimino)ethyl)-6a,8a,10,10-tetramethyl-1,2,6a,6b,7,8,8a,8b,11a,12,12a,12b-dodecyl-4H-naphtho[2',1':4,5]indeno[1,2-d][1,3]dioxan-4-one oxime

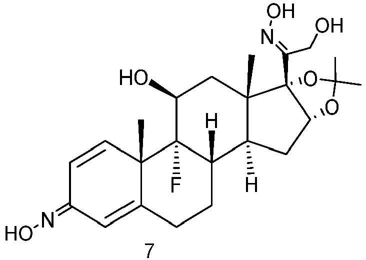

Compound 7 was prepared according to the methods shown in Synthesis Routes I and III in the general synthetic methods above using appropriate starting materials and separated to give (3*Z*)-7 and (3*E*)-7.

Compound 3 (*Z*)-7: ¹H-NMR (600MHz, d₆-DMSO) δ_{H} 0.731(m, 1H), 0.877-0.852 (m, 4H), 1.233(s, 6H), 1.287-1.198 (m,1H), 1.347(s, 3H), 1.611-1.597 (m,1H), 1.783-1.774(m, 2H), 1.998-1.972(m, 2H), 2.798(m, 2H), 3.986(m, 1H), 4.174-4.163(m, 1H), 4.285-4.273(m, 2H), 4.411-4.402(m, 1H), 4.587-4.559(m, 1H), 6.118-6.097(m, 1H), 6.361-6.333(d, 1H), 6.442(s, 1H), 10.574(s, 1H), 10.799(s, 1H); ¹³C(600MHz, d₆-DMSO) δ_{C} 18.34, 23.62, 26.57, 33.86, 33.92, 34.37, 39.94, 43.51, 43.69, 47.08, 50.92, 55.76, 70.83, 83.49, 96.91, 100.24, 104.82, 121.62, 123.13, 141.72, 148.75, 159.73, 161.49 ; Calculated value 465.3 Experimental value 465.2 for LRMS(ES+)[ C₂₃H₃₃F₂N₂O₆+H].

Compound 3 (*E*)-7: ¹H-NMR (600MHz, d₆-DMSO) δ_{H} 0.728(m, 1H), 0.874-0.833 (m, 4H), 1.234(s, 6H), 1.256-1.121 (m,1H), 1.326(s, 3H), 1.609-1.593 (m, 1H), 1.762-1.723(m, 2H), 2.003-1.965(m, 2H), 2.788(m, 2H), 3.979(m, 1H), 4.154-4.123(m, 1H), 4.269-4.253(m, 2H), 4.407-4.403(m, 1H), 4.591-4.570(m, 1H), 5.706(s, 1H), 6.513-6.504(d, 1H), 6.779-6.759(m, 1H), 10.522(s, 1H), 10.793(s, 1H); ¹³C(600MHz, d₆-DMSO) δ_{C} 18.33, 23.64, 26.56, 33.85, 33.94, 34.38, 39.93, 43.47, 43.64, 47.03, 50.93, 55.75, 70.82, 83.56, 96.94, 100.23, 112.25, 113.72, 121.63, 145.97, 149.09, 156.88, 159.74 ; Calculated value 465.3 Experimental value 465.2 for LRMS(ES+)[ C₂₃H₃₃F₂N₂O₆+H].

### Example 8

### (2S,6aS,6bR,7S,8aS,8bR,11aR,12aS,12bS)-2,6b-difluoro-7-hydroxy-8b-((E)-2-hydroxy-1-(hydroxyimino)ethyl)-6a,8a,10,10-tetramethyl-1,2,6a,6b,7,8,8a,8b,11a,12,12a,12b-dodecahydro-4H-naphtho[2',1':4,5]indeno[1,2-d][1,3]dioxol-4-one oxime

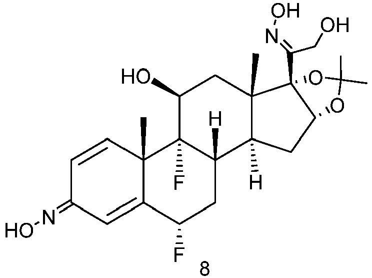

Compound 8 was prepared according to the methods shown in Synthesis Routes I and III in the general synthetic methods above using appropriate starting materials and separated to give (3*Z*)-8 and (3*E*)-8.

Compound 3(*Z*)-8: ¹H-NMR (600MHz, d₆-DMSO) δ_{H} 0.702(m, 1H), 0.876-0.853 (m, 4H), 1.235(s, 6H), 1.258-1.121 (m,1H), 1.331(s, 3H), 1.613-1.598(m,1H), 1.782-1.775(m, 2H), 1.871-1.859(m, 1H), 1.998-1.972(m, 1H), 3.983(m, 1H), 4.093-4.087(m, 1H), 4.172-4.165(m, 1H), 4.289-4.276(m, 2H), 4.404(m, 1H), 4.583-4.563(m, 1H), 6.124-6.102(m, 1H), 6.364-6.341(d, 1H), 6.573(s, 1H), 10.576(s, 1H), 10.801(s, 1H); ¹³C(600MHz, d₆-DMSO) δ_{C} 18.35, 23.64, 26.58, 33.87, 34.38, 39.97, 43.59, 43.73, 47.11, 50.94, 55.75, 70.85, 83.47, 88.87, 96.94, 100.25, 104.83, 121.60, 123.14, 141.73, 148.76, 159.72, 161.03 ; Calculated value 483.2 Experimental value 483.3 for LRMS(ES+)[ C₂₄H₃₂F₂N₂O₆+H].

Compound 3(*E*)-8: ¹H-NMR (600MHz, d₆-DMSO) δ_{H} 0.703(m, 1H), 0.874-0.851 (m, 4H), 1.234(s, 6H), 1.256-1.124 (m,1H), 1.328(s, 3H), 1.610-1.595 (m, 1H), 1.771-1.765(m, 2H), 1.872-1.861(m, 1H), 2.002-1.974(m, 1H), 3.977(m, 1H), 4.091-4.085(m, 1H), 4.152-4.126(m, 1H), 4.271-4.254(m, 2H), 4.406 (m, 1H), 4.587-4.569(m, 1H), 5.784(s, 1H), 6.521-6.510(d, 1H), 6.775-6.761(m, 1H), 10.523(s, 1H), 10.789(s, 1H); ¹³C(600MHz, d₆-DMSO) δ_{C} 18.34, 23.65, 26.57, 33.86, 34.37, 39.95, 43.58, 43.75, 47.08, 50.95, 55.76, 70.83, 83.54, 96.96, 100.24, 112.24, 113.75, 121.62, 145.96, 149.11, 156.87, 159.72 ; Calculated value 483.2 Experimental value 483.3 for LRMS(ES+)[ C₂₄H₃₂F₂N₂O₆+H].

### Example 9

### (6S,8S,9S,10R,11S,13S,14S,17R,E)-17-((E)-1-(ethoxysimino)-2-hydroxyethyl)-11,17-dihydroxy-6,10,13-trimethyl-6,7,8,9,10,11,12,13,14,15,16,17-dodecahydro-3H-cyclopentane[a]phenanthrene-3-ketoO-ethyl oxime

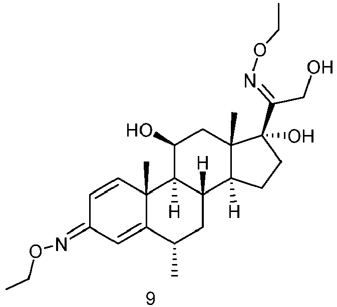

Compound 9 was prepared according to the methods shown in Synthesis Routes I and IV in the general synthetic methods above using appropriate starting materials and separated to give (3*Z*)-9 and (3*E*)-9.

Compound 3 (*Z*)-9: ¹H-NMR (600MHz, d₆-DMSO) δ_{H} 0.629(m, 1H), 0.870-0.835 (m, 4H), 0.987-0.975(m, 6H), 1.051-1.035 (d, 3H), 1.254-1.119 (m,1H), 1.323(s, 3H), 1.396-1.354 (m, 1H), 1.604-1.590 (m, 2H), 1.758-1.714(m, 2H), 1.995-1.969(m, 2H), 2.636-2.536(m, 1H), 2.734(m, 1H), 3.882-3.754(m, 4H), 4.164-4.132(m, 1H), 4.274-4.225(m, 2H), 4.406-4.397(m, 1H), 4.589-4.560(m, 1H), 4.807(s, 1H), 6.116-6.087(m, 1H), 6.354-6.329(d, 1H), 6.446(s, 1H),; ¹³C(600MHz, d₆-DMSO) δ_{C} 13.9, 18.26, 18.64, 23.47, 23.62, 31.73, 32.75, 32.84, 39.93, 43.52, 43.66, 47.09, 50.95, 55.79, 56.97, 66.93, 67.02, 67.75, 85.69, 104.84, 121.61, 141.76, 148.73, 159.74, 161.48 ; Calculated value 461.3 Experimental value 461.4 for LRMS(ES+)[ C₂₆H₄₀N₂O₅+H].

Compound 3 (*E*)-9: ¹H-NMR (600MHz, d₆-DMSO) δ_{H} 0.631-0.624(m, 1H), 0.873-0.835 (m, 4H), 0.985-0.974(m, 6H), 1.035-1.028 (d, 3H), 1.253-1.121 (m,1H), 1.326(s, 3H), 1.365-1.351 (m, 1H), 1.604-1.592 (m, 2H), 1.760-1.713(m, 2H), 2.002-1.954(m, 2H), 2.516(m, 1H), 2.735(m, 1H), 3.796-3.728(m, 4H), 4.157-4.128(m, 1H), 4.269-4.235(m, 2H), 4.407-4.401(m, 1H), 4.590-4.571(m, 1H), 4.804(s, 1H), 5.702(s, 1H), 6.517-6.506(d, 1H), 6.801-6.762(m, 1H); ¹³C(600MHz, d₆-DMSO) δ_{C} 14.01, 18.24, 18.58, 23.33, 23.49, 31.75, 32.66, 32.83, 39.96, 43.41, 43.64, 47.02, 50.93, 55.85, 56.70, 67.72, 85.73, 112.21, 113.76, 145.94, 149.07, 156.83, 159.74 ; Calculated value 461.3 Experimental value 461.4 for LRMS(ES+)[ C₂₆H₄₀N₂O₅+H].

### Example 10

### (8S,9S,10R,11S,13S,14S,16R,17S)-11-hydroxy-17-(2-hydroxyacetyl)-10,13,16-trimethyl-6,7,8,9,10,11,12,13,14,15,16,17-dodecahydro-3H-cyclopentane[a]phenanthrene-3-one

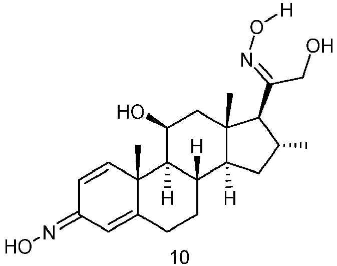

Compound 10 was prepared according to the methods shown in Synthesis Route I in the general synthetic methods above using appropriate starting materials and separated to give (3*Z*)-10 and (3*E*)-10.

Compound 3 (*Z*)-10: ¹H-NMR (600MHz, d₆-DMSO) δ_{H} 0.630(m, 1H), 0.870-0.832 (m, 4H), 0.989-0.975(s, 3H), 1.254-1.126 (m,1H), 1.327(s, 3H), 1.602-1.593 (m, 2H), 1.755-1.716(m, 3H), 1.896-1.874(m, 1H), 2.003 -1.977(m, 2H), 2.606-2.528(m, 2H), 4.163-4.142(m, 1H), 4.273-4.231(m, 2H), 4.405-4.396(m, 1H), 4.591-4.575(m, 1H), 6.113-6.087(m, 1H), 6.354-6.329(m, 1H), 6.453(s, 1H), 10.567(s, 1H), 10.797(s, 1H); ¹³C(600MHz, d₆-DMSO) δ_{C} 17.21, 18.32, 23.51, 31.42, 31.96, 38.95, 39.71, 40.03, 42.97, 43.92, 45.88, 49.73, 56.81, 56.89, 67.76, 85.67, 104.83, 121.59, 141.73, 148.76, 159.72, 161.47 ; Calculated value 389.3 Experimental value 389.2 for LRMS(ES+)[ C₂₂H₃₂N₂O₄+H].

Compound 3 (*E*)-10: ¹H-NMR (600MHz, d₆-DMSO) δ_{H} 0.628(m, 1H), 0.873-0.834(m, 4H), 0.988-0.976(s, 3H), 1.253-1.121 (m,1H), 1.327(s, 3H), 1.606-1.594 (m, 2H), 1.764-1.712(m, 3H), 1.894-1.873(m, 1H),1.998-1.962(m, 2H), 2.597-2.485(m, 2H), 4.155-4.134(m, 1H), 4.267-4.231(m, 2H), 4.408-4.403(m, 1H), 4.588-4.572(m, 1H), 5.704(s, 1H), 6.511-6.503(d, 1H), 6.778-6.764(m, 1H), 10.522(s, 1H), 10.793(s, 1H); ¹³C(600MHz, d₆-DMSO) δ_{C} 17.25, 18.26, 23.37, 31.39, 31.87, 38.93, 39.69, 40.01, 42.92, 43.89, 45.86, 49.71, 56.76, 56.85, 67.70, 85.64, 112.19, 113.78, 145.94, 149.11, 156.87, 159.82 ; Calculated value 389.3 Experimental value 389.2 for LRMS(ES+)[ C₂₂H₃₂N₂O₄+H].

### Example 11

### (2E)-2-(ethoxysilane)-2-((8S,9S,10R,11S,13S,14S,16R,17R)-3-(ethoxysilane)-11,17-dihydroxy-10,13,16-trimethyl-6,7,8,9,10,11,12,13,14,15,16,17-dodecahydro-3H-cyclopentanone[a]phenanthrene-17-yl) ethyl acetate

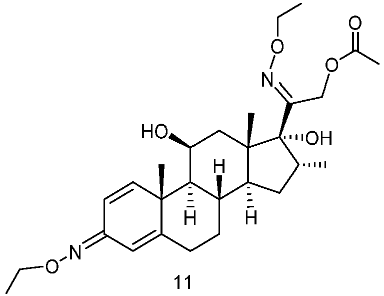

Compound 11 was prepared according to the methods shown in Synthesis Routes I and IV in the general synthetic methods above using appropriate starting materials and separated to give (3*Z*)-11 and (3*E*)-11.

Compound 3(*Z*)-11: ¹H-NMR (600MHz, d₆-DMSO) δ_{H} 0.641(m, 1H), 0.877-0.841 (m, 4H), 0.992-0.975(s, 9H), 1.254-1.126 (m,1H), 1.327(s, 3H), 1.602-1.593 (m, 2H), 1.755-1.716(m, 3H), 2.003 -1.977(m, 2H), 2.606-2.528(m, 2H), 2.688(s, 3H), 3.882-3.754(m, 4H), 4.163-4.142(m, 1H), 4.273-4.231(m, 2H), 4.405-4.396(m, 1H), 4.806(s, 1H), 6.113-6.087(m, 1H), 6.354-6.329(m, 1H), 6.453(s, 1H); ¹³C(600MHz, d₆-DMSO) δ_{C} 13.56, 17.21, 18.33, 23.53, 30.35, 31.63, 32.58, 36.83, 39.98, 42.36, 43.62, 46.11, 50.83, 56.89, 56.93, 67.74, 69.81, 69.8488.87,104.85,121.54,141.76,148.74,159.71,161.45,169.88 ; Calculated value 503.3 Experimental value 503.2 for LRMS(ES+)[ C₂₈H₄₂N₂O₆+H].

Compound 3(*E*)-11: ¹H-NMR (600MHz, d₆-DMSO) δ_{H} 0.633(m, 1H), 0.875-0.837(m, 4H), 0.987-0.979(s, 9H), 1.2543-1.120 (m,1H), 1.329(s, 3H), 1.604-1.592 (m, 2H), 1.761-1.718(m, 3H), 1.997-1.965(m, 2H), 2.596-2.483(m, 2H), 2.687(s, 3H), 3.876-3.752(m, 4H), 4.154-4.132(m, 1H), 4.264-4.230(m, 2H), 4.404-4.398(m, 1H), 4.811(s, 1H), 5.706(s, 1H), 6.509-6.501(d, 1H), 6.776-6.763(m, 1H); ¹³C(600MHz, d₆-DMSO) δ_{C} 13.55, 17.23, 18.34, 23.51, 30.39, 31.67, 32.56, 36.76, 39.96, 42.37, 43.69,46.13, 50.79, 56.86, 56.96, 67.72, 69.79, 69.83, 88.84, 112.21, 113.76, 145.92, 149.15, 156.86, 159.76, 169.87 ; Calculated value 503.3 Experimental value 503.2 for LRMS(ES+)[ C₂₈H₄₂N₂O₆+H].

### Example 12

### (2E)-2-((8S,9R,10S,11S,13S,14S,16S,17R)-9-chloro-11,17-dihydroxy-3-(hydroxyimino)-10,13,16-trimethyl-6,7,8,9,10,11,12,13,14,15,16,17-dodecahydro-3H-cyclopentane[a]phenanthrene-17-yl)-2-(hydroxyimino) ethyl propionate

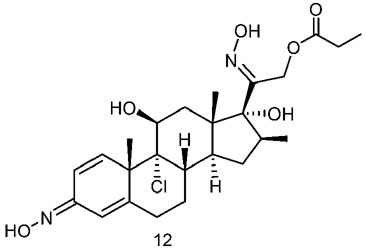

Compound 12 was prepared according to the methods shown in Synthesis Routes I and II in the general synthetic methods above using appropriate starting materials and separated to give (3*Z*)-12 and (3*E*)-12.

Compound 3(*Z*)-12: ¹H-NMR (600MHz, d₆-DMSO) δ_{H} 0.629(m, 1H), 0.872-0.833 (m, 7H), 1.249-1.117 (m, 4H), 1.326(s, 3H), 1.601-1.597 (m, 1H), 1.754-1.718(m, 3H), 2.002 - 1.976(m, 2H), 2.601-2.522(m, 4H), 4.159-4.134(m, 1H), 4.274-4.229(m, 2H), 4.402-4.398(m, 1H), 4.811(s, 1H), 6.109-6.085(m, 1H), 6.353-6.321(d, 1H), 6.452(s, 1H), 10.574(s, 1H), 10.792(s, 1H); ¹³C(600MHz, d₆-DMSO) δ_{C} 10.26, 17.27, 18.24, 23.58, 27.29, 27.36, 31.76, 32.44, 38.90, 39.96, 43.55, 43.67, 47.04, 50.91, 55.76, 56.93, 67.77, 88.96, 104.85, 121.63, 141.76, 148.73, 159.74, 161.46, 171.57 ; Calculated value 496.2 Experimental value 496.3 for LRMS(ES+)[ C₂₅H₃₅FN₂O₆+H].

Compound 3(*E*)-12: ¹H-NMR (600MHz, d₆-DMSO) δ_{H} 0.631(m, 1H), 0.871-0.837(m, 7H), 1.252-1.121 (m, 4H), 1.322(s, 3H), 1.603-1.597 (m, 1H), 1.761-1.723(m, 3H), 1.998-1.967(m, 2H), 2.596-2.486(m, 4H), 4.154-4.133(m, 1H), 4.272-4.234(m, 2H), 4.406-4.397(m, 1H), 4.809(s, 1H), 5.698(s, 1H), 6.508-6.497(d, 1H), 6.772-6.760(m, 1H), 10.528(s, 1H), 10.791(s, 1H); ¹³C(600MHz, d₆-DMSO) δ_{C} 10.24, 17.25, 18.23, 23.39, 27.31, 27.40, 31.75, 32.48, 38.89, 39.93, 43.54, 43.67, 47.04, 50.93, 55.77, 56.71, 67.68, 88.92, 112.21, 113.78, 145.96, 149.11, 156.82, 159.77, 171.52 ; Calculated value 496.2 Experimental value 496.3 for LRMS(ES+)[ C₂₅H₃₅FN₂O₆+H].

### Example 13

### (6aR,6bS,7S,8aS,8bR,11aR,12aS,12bS)-7-hydroxy-8b-((E)-2-hydroxy-1-(hydroxyimino)ethyl)-6a,8a-dimethyl-10-propyl-1,2,6a,6b,7,8,8a,8b,11 a,12,12a,12b-dodecahydro-4H-naphtho[2',1':4,5]indeno[1,2-d][1,3]dioxol-4-one oxime

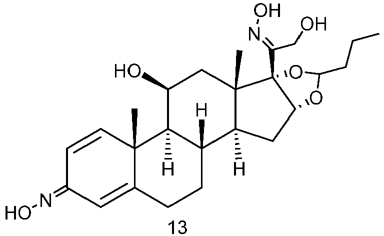

Compound 13 was prepared according to the methods shown in Synthesis Routes I and III in the general synthetic methods above using appropriate starting materials and separated to give (3*Z*)-13 and (3*E*)-13.

Compound 3(*Z*)-13: ¹H-NMR (600MHz, d₆-DMSO) δ_{H} 0.637(m, 1H), 0.873-0.858 (m, 4H), 0.893-0.882(m, 3H), 1.233(s, 2H), 1.272-1.125 (m, 1H), 1.347(s, 3H), 1.402(m, 1H), 1.613-1.602 (m, 3H), 1.781-1.779(m, 2H), 1.994-1.961(m, 2H), 2.679-2.657(m, 2H), 3.986(m, 1H), 4.170-4.168(m, 1H), 4.283-4.269(m, 2H), 4.407-4.404(m, 1H), 4.583-4.563(m, 1H), 5.396(m, 1H), 6.118-6.097(m, 1H), 6.361-6.333(d, 1H), 6.442(s, 1H), 10.574(s, 1H), 10.799(s, 1H); ¹³C(600MHz, d₆-DMSO) δ_{C} 13.22, 14.57, 18.36, 23.55, 33.88, 33.91, 34.36, 36.69, 42.85, 43.54, 43.61, 46.97, 52.38, 55.76, 56.98, 70.21, 89.97, 96.91, 103.62 104.83, 120.31, 141.75, 148.73, 159.76, 161.46 ; Calculated value 461.3 Experimental value 461.4 for LRMS(ES+)[ C₂₅H₃₆N₂O₆+H].

Compound 3(*E*)-13: ¹H-NMR (600MHz, d₆-DMSO) δ_{H} 0.635(m, 1H), 0.876-0.842(m, 4H), 0.891-0.884(m, 3H), 1.231(s, 2H), 1.269-1.123 (m, 1H), 1.331(s, 3H), 1.398(m, 1H), 1.7-1.594(m, 3H), 1.767-1.728(m, 2H), 2.001-1.967(m, 2H), 2.749(m, 2H), 3.982(m, 1H), 4.163-4.139(m, 1H), 4.271-4.262(m, 2H), 4.409-4.405(m, 1H), 4.590-4.568(m, 1H), 5.704(s, 1H), 6.517-6.501(d, 1H), 6.778-6.757(m, 1H), 10.528(s, 1H), 10.796(s, 1H); ¹³C(600MHz, d₆-DMSO) δ_{C} 13.26, 14.55, 18.34, 23.57, 33.87, 33.89, 34.35, 36.72, 42.83, 43.49, 43.63, 46.94, 52.37, 55.74, 56.97, 70.24, 89.95, 96.93, 103.65, 112.29, 113.74, 120.33, 145.94, 149.03, 156.86, 159.73 ; Calculated value 461.3 Experimental value 461.4 for LRMS(ES+)[ C₂₅H₃₆N₂O₆+H].

### Example 14

### (2E)-2-((8S,9R,10S,11S,13S,14S,16R,17R)-9-fluoro-11,16,17-trihydroxy-3-(hydroxyimino)-10,13-dimethyl-6,7,8,9,10,11,12,13,14,15,16,17-dodecahydro-3H-cyclopentane[a]phenanthrene-17-yl)-2-(hydroxyimino) ethyl propionate

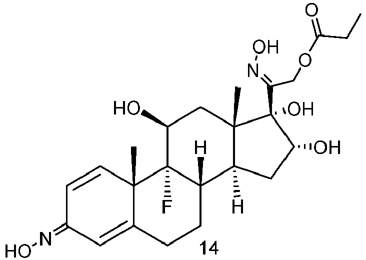

Compound 14 was prepared according to the methods shown in Synthesis Routes I and II in the general synthetic methods above using appropriate starting materials and separated to give (3*Z*)-14 and (3*E*)-14.

Compound 3(*Z*)-14: ¹H-NMR (600MHz, d₆-DMSO) δ_{H} 0.728(m, 1H), 0.879-0.854 (m, 4H), 1.211-1.199(m, 3H), 1.284-1.187 (m,1H), 1.347(s, 3H), 1.611-1.597 (m, 1H), 1.783-1.774(m, 2H), 1.998-1.972(m, 2H), 2.399-2.367(m, 2H), 2.798(m, 2H), 3.873(m, 1H), 4.161-4.148(m, 2H), 4.376-4.363(m, 1H), 4.411-4.402(m, 1H), 5.223(s, 1H), 5.416(m, 1H), 6.114-6.093(m, 1H), 6.358-6.331(d, 1H), 6.438(s, 1H), 10.571(s, 1H), 10.795(s, 1H); ¹³C(600MHz, d₆-DMSO) δ_{C} 10.32, 18.21, 23.32, 23.57, 27.46, 33.89, 33.94, 34.29, 39.91, 43.50, 43.71, 47.08, 50.81, 56.69, 70.81, 82.34, 92.73, 100.21, 104.84, 121.60, 141.71, 148.77, 159.72, 161.45 ; Calculated value 481.2 Experimental value 481.3 for LRMS(ES+)[ C₂₄H₃₃F₁N₂O₇+H].

Compound 3(*E*)-14: ¹H-NMR (600MHz, d₆-DMSO) δ_{H} 0.731(m, 1H), 0.872-0.835(m, 4H), 1.208-1.198(m, 3H), 1.253-1.119 (m,1H), 1.329(s, 3H), 1.607-1.592(m, 1H), 1.765-1.738(m, 2H), 2.001-1.969(m, 2H),2.334-2.359(m, 2H), 2.787(m, 2H), 3.897(m, 1H), 4.154-4.123(m, 2H), 4.272-4.259(m, 1H), 4.406-4.401(m, 1H), 5.219(s, 1H), 5.408(m, 1H), 5.704(s, 1H), 6.511-6.502(d, 1H), 6.774-6.758(m, 1H), 10.523(s, 1H), 10.798(s, 1H); ¹³C(600MHz, d₆-DMSO) δ_{C} 10.29, 18.24, 23.34, 23.56, 27.45, 33.88, 33.95, 34.31, 39.92, 43.49, 43.69, 47.05, 50.83, 56.68, 70.79, 82.36, 92.72, 100.22, 112.23, 113.74, 145.95, 149.07, 156.86, 159.73 ; Calculated value 481.2 Experimental value 481.3 for LRMS(ES+)[ C₂₄H₃₃F₁N₂O₇+H].

### Example 15

### (8S,9R,10S,11S,13S,14S,16S,17R)-9-fluoro-11-hydroxy-17-((E)-2-hydroxy-1-(hydroxyimino)ethyl)-3-(hydroxyimino)-10,13,16-trimethyl-6,7,8,9,10,11,12,13,14,15,16,17-dodecahydro-3H-cyclopentaphenanthrene-17-yl butyrate

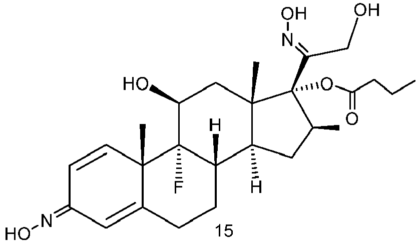

Compound 15 was prepared according to the methods shown in Synthesis Routes I and II in the general synthetic methods above using appropriate starting materials and separated to give (3*Z*)-15 and (3*E*)-15.

Compound 3(*Z*)-15: ¹H-NMR (600MHz, d₆-DMSO) δ_{H} 0.631(m, 1H), 0.872-0.833 (m, 7H), 1.109-0.998(m, 3H), 1.249-1.119 (m, 1H), 1.331(s, 3H), 1.601-1.597 (m, 1H), 1.665-1.621(m, 2H), 1.754-1.718(m, 2H), 1.998 -1.975(m, 2H), 2.136-1.109(m, 1H), 2.322-2.317(m, 2H), 2.603-2.524(m, 2H), 4.145-4.122(m, 1H), 4.275-4.227(m, 2H), 4.403-4.396(m, 1H), 4.589-4.560(m, 1H), 6.113-6.086(m, 1H), 6.359-6.324(d, 1H), 6.448(s, 1H), 10.578(s, 1H), 10.799(s, 1H); ¹³C(600MHz, d₆-DMSO) δ_{C} 14.11, 17.21, 18.25, 18.46, 23.52, 23.66, 31.62, 32.49, 32.79, 36.32, 40.11, 43.46, 43.62, 47.87, 51.12, 55.72, 67.77, 88.91, 100.01, 104.83, 121.67, 141.72, 148.75, 159.71, 161.48, 172.13 ; Calculated value 493.3 Experimental value 493.2 for LRMS(ES+)[ C₂₆H₃₇FN₂O₆+H].

Compound 3(*E*)-15: ¹H-NMR (600MHz, d₆-DMSO) δ_{H} 0.629(m, 1H), 0.873-0.834(m, 7H), 1.107-0.997(m, 3H), 1.246-1.117 (m, 1H), 1.322(s, 3H), 1.602-1.599 (m, 1H), 1.661-1.618(m, 2H), 1.755-1.721(m, 2H), 1.996-1.972(m, 2H), 2.133-1.107(m, 1H), 2.317-2.311(m, 2H), 2.597-2.493(m, 2H), 4.142-4.120(m, 1H), 4.272-4.225(m, 2H), 4.404-4.395(m, 1H), 4.588-4.571(m, 1H), 5.683(s, 1H), 6.507-6.494(d, 1H), 6.770-6.758(m, 1H), 10.526(s, 1H), 10.801 (s, 1H); ¹³C(600MHz, d₆-DMSO) δ_{C} 14.09, 17.24, 18.23, 18.44, 23.41, 23.64, 31.64, 32.47, 32.75, 36.33, 40.08, 43.42, 43.63, 47.84, 51.11, 55.74, 67.69, 88.90, 99.98, 112.19, 113.75, 145.93, 149.13, 156.83, 159.74, 172.11 ; Calculated value 493.3 Experimental value 493.2 for LRMS(ES+)[ C₂₆H₃₇FN₂O₆+H].

### Example 16

### (8S,9S,10R,11S,13S,14S,17R)-11-hydroxy-3-(hydroxyimino)-17-((E)-1-(hydroxyimino)ethyl)-10,13-dimethyl-6,7,8,9,10,11,12,13,14,15,16, 17-dodecahydro-3H-cyclopentane[a]phenanthrene-17-yl propionate

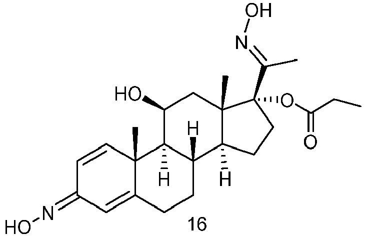

Compound 16 was prepared according to the methods shown in Synthesis Routes I and II in the general synthetic methods above using appropriate starting materials and separated to give (3*Z*)-16 and (3*E*)-16.

Compound 3(*Z*)-16: ¹H-NMR (600MHz, d₆-DMSO) δ_{H} 0.628(m, 1H), 0.868-0.835 (m, 4H), 1.226-1.213(m, 3H), 1.253-1.118(m,1H), 1.325(s, 3H), 1.398-1.364(m, 1H), 1.606-1.590(m, 2H), 1.760-1.715(m, 2H), 1.998-1.972(m, 2H), 2.339-2.297(m, 2H), 2.606-2.527(m, 2H), 2.755(m, 1H), 3.037(s, 3H), 4.273-4.224(m, 1H), 4.402-4.394(m, 1H), 6.118-6.086(m, 1H), 6.355-6.329(d, 1H), 6.447(s, 1H), 10.575(s, 1H), 10.794(s, 1H); ¹³C(600MHz, d₆-DMSO) δ_{C} 9.97, 10.92, 18.34, 23.56, 23.62, 28.06, 31.67, 32.50, 32.72, 39.98, 43.58, 43.69, 46.24, 50.94, 56.95, 67.72, 95.58, 104.84, 121.59, 141.72, 148.77, 159.71, 161.48, 173.36 ; Calculated value 431.2 Experimental value 431.3 for LRMS(ES+)[ C₂₄H₃₄N₂O₅+H].

Compound 3(*E*)-16: ¹H-NMR (600MHz, d₆-DMSO) δ_{H} 0.629(m, 1H), 0.870-0.834(m, 4H), 1.224-1.210(m, 3H), 1.250-1.121(m,1H), 1.322(s, 3H), 1.375-1.363(m, 1H), 1.603-1.595(m, 2H), 1.755-1.709(m, 2H), 2.002-1.975(m, 2H), 2.331-2.295(m, 2H), 2.603-2.532(m, 2H), 2.745(m, 1H), 3.035(s, 3H), 4.271-4.228(m, 2H), 4.403-4.398(m, 1H), 5.704(s, 1H), 6.515-6.501(d, 1H), 6.776-6.758(m, 1H), 10.520(s, 1H), 10.791(s, 1H); ¹³C(600MHz, d₆-DMSO) δ_{C} 9.98, 10.89, 18.33, 23.46, 23.61, 28.04, 31.63, 32.46, 32.84, 39.97, 43.54, 43.66, 46.20, 50.92, 56.97, 67.74, 95.57, 112.19, 113.73, 145.97, 149.08, 156.85, 159.71, 173.34 ; Calculated value 431.2 Experimental value 431.3 for LRMS(ES+)[ C₂₄H₃₄N₂O₅+H].

### Example 17

### (6S,8S,9S,10R,11S,13S,14S,17R,E)-11-hydroxy-17-((E)-2-hydroxy-1-(hydroxyimino)ethyl)-3-(hydroxyimino)-6,10,13-trimethyl-6,7,8,9,10,11,12,13,14,15,16,17-dodecahydro-3H-cyclopentane[a]phenanthrene-17-yl valerate

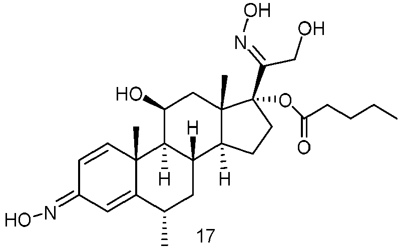

Compound 17 was prepared according to the methods shown in Synthesis Routes I and II in the general synthetic methods above using appropriate starting materials and separated to give (3*Z*)-17 and (3*E*)-17.

Compound 3(*Z*)-17: ¹H-NMR (600MHz, d₆-DMSO) δ_{H} 0.628(m, 1H), 0.869-0.833(m, 4H), 0.942-0.921(m, 3H), 1.053-1.033(d, 3H), 1.253-1.117(m,1H), 1.325(s, 3H), 1.387-1.334(m, 2H), 1.498-1.424(m, 1H), 1.604-1.588(m, 2H), 1.755-1.702(m, 4H), 1.996-1.971(m, 2H), 2.429-2.402(m, 2H), 2.634-2.532(m, 1H), 2.789(m, 1H), 4.160-4.129(m, 1H), 4.272-4.227(m, 2H), 4.403-4.396(m, 1H), 4.591-4.562(m, 1H), 6.115-6.089(m, 1H), 6.353-6.331(d, 1H), 6.446(s, 1H), 10.575(s, 1H), 10.794(s, 1H); ¹³C(600MHz, d₆-DMSO) δ_{C} 13.92, 18.34, 18.61, 22.37, 23.57, 23.61, 27.59, 31.65, 32.57, 32.74, 33.98, 40.13, 43.52, 43.69, 45.02, 50.99, 56.32, 56.95, 67.63, 89.34, 104.83, 121.60, 141.72, 148.75, 159.73, 161.48, 173.24 ; Calculated value 489.3 Experimental value 489.4 for LRMS (ES+) [C₂₇H₄₀N₂O₆+H].

Compound 3(*E*)-17: ¹H-NMR (600MHz, d₆-DMSO) δ_{H} 0.623(m, 1H), 0.874-0.846(m, 4H), 0.940-0.918(m, 3H), 1.033-1.018(d, 3H), 1.252-1.120(m,1H), 1.326(s, 3H), 1.385-1.327(m, 2H), 1.495-1.443(m, 1H), 1.603-1.592(m, 2H), 1.762-1.701(m, 4H), 2.002-1.958(m, 2H), 2.411-2.387(m, 2H), 2.514(m, 1H), 2.786(m, 1H), 4.156-4.125(m, 1H), 4.262-4.230(m, 2H), 4.410-4.405(m, 1H), 4.590-4.568(m, 1H), 5.704(s, 1H), 6.516-6.500(d, 1H), 6.781-6.758(m, 1H), 10.523(s, 1H), 10.795(s, 1H); ¹³C(600MHz, d₆-DMSO) δ_{C} 13.91, 18.29, 18.60, 22.35, 23.37, 23.52, 27.61, 31.69, 32.58, 32.75, 33.96, 40.14, 43.51, 43.67, 45.03, 50.98, 56.33, 56.96, 67.71, 89.28, 112.21, 113.76, 145.94, 149.08, 156.85, 159.73, 173.22 ; Calculated value 489.3 Experimental value 489.4 for LRMS (ES+) [C₂₇H₄₀N₂O₆+H].

The compounds prepared in Examples 1-17 have strong anti-gout efficacy. The specific experiments are as follows. Among them, the compounds of the Examples used in the following experiments are mixtures of two configurations of *cis-* and *trans-* oxime group at 3-position of the compounds prepared in each Example in an equal ratio (1:1).

### Experiment 1

Gout is a disease unique to human, and gouty arthritis is an inflammatory reaction caused by the deposition of monosodium urate (MSU) in local joint tissues. Since purine metabolism pathways in some mammals such as mice and rabbits are different from those in humans, it is impossible to construct a gouty arthritis model by the method of interfering with purine metabolism disorders, so the most direct method for causing gouty arthritis is to directly inject MSU into the joint cavity of the model animal. The model is only used for simulating local joint inflammation of gout, is suitable for observation of local arthritis symptoms, is a common method for screening anti-inflammatory effects of gout drugs at present, and has the advantages of stability, easy operation and the like. (Reference: Li Na, Hua Long, and Yuan Hui. Research progress of gout animal models [J]. Medical theory and practice, 2016(24).

The anti-gout effect of the compound prepared in the above Example was detected by using a rat paw swelling model induced by monosodium urate (MSU), and the control sample was methylprednisolone.

Experimental method: MSU was used as an inflammatory agent, injected subcutaneously into the hind paws of rats to cause paw swelling, and then the volume of the paws was measured, and the swelling rates and swelling inhibition rates of the treatment group and the control group were compared. On the day of the experiment, the volume of right hind paw (ml) of each rat was measured firstly, and then after the onset of inflammation, the volume of right hind paw was measured so that the difference of volume of paw before and after inflammation was swelling degree, and the swelling inhibition rate of paw (swelling inhibition rate %=(C-T)/C* 100%); C= swelling rate of the control group %, and T= swelling rate of the treatment group) was calculated. This method is one of the commonly used methods for screening anti-inflammatory effects of gout drugs, and the model is stable and easy to operate.
(1) The dosage is positively correlated with the anti-gout effect, and the specific experimental results are as follows: 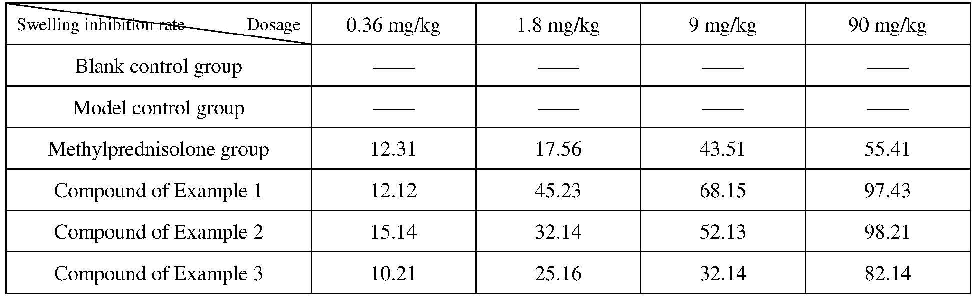 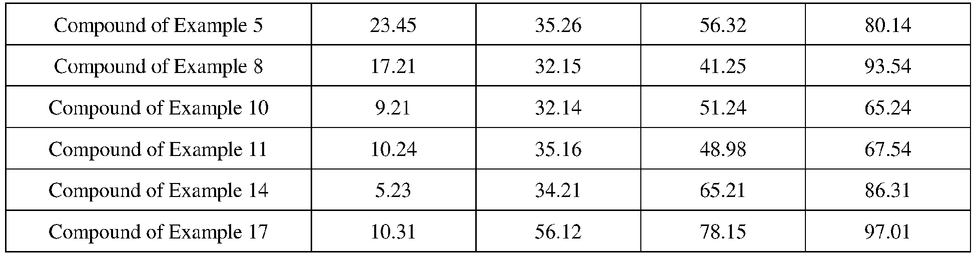
(2) One week before the subcutaneous injection of MSU into the feet and soles of the rats, the rats were given different doses of the test drugs, and the drugs were given by gavage once a day, for seven consecutive days. The circumference of the right hind paw was marked and measured before the last administration, and the average value of the two measurements was taken as the standard. 1 h after the last administration, animals in other groups were given 50 µL of 2% MSU in addition to the blank control group, and the paw circumference was measured 1 h after the onset of inflammation. The results of administering to mice 90 mg/kg methylprednisolone, and the compound of the above Example were as follows:

| | Mean volume of paw before administration (ml) | Mean volume of paw after administration (ml) | Swelling value (ml) | Swelling degree (%) | Swelling inhibition rate (%) |
|---|---|---|---|---|---|
| Blank control group | 1.21 | 1.22 | 0.01 | 0.83 | - |
| Model control group | 1.23 | 5.28 | 4.05 | 329.27 | - |
| Methylprednisolone group | 1.23 | 3.1 | 1.87 | 152.03 | 53.83 |
| Compound of Example 1 | 1.18 | 1.19 | 0.01 | 0.85 | 99.74 |
| Compound of Example 2 | 1.24 | 1.28 | 0.04 | 3.23 | 99.02 |
| Compound of Example 3 | 1.2 | 1.97 | 0.77 | 64.17 | 80.51 |
| Compound of Example 5 | 1.22 | 1.94 | 0.72 | 59.02 | 82.08 |
| Compound of Example 8 | 1.23 | 1.56 | 0.33 | 26.83 | 91.85 |
| Compound of Example 10 | 1.21 | 2.45 | 1.24 | 102.48 | 68.88 |
| Compound of Example 11 | 1.22 | 2.64 | 1.42 | 116.39 | 64.65 |
| Compound of Example 14 | 1.18 | 1.74 | 0.56 | 47.46 | 85.59 |
| Compound of Example 17 | 1.18 | 1.32 | 0.14 | 11.86 | 96.4 |

In this experiment, SD rats were consecutively administered different doses of the compounds one week before the subcutaneous injection of MSU into the soles. The test results showed that the swelling inhibition rate (%) of the compounds of the above Examples was in the range of 64.65% to 99.74%, and within the safe dose, the anti-gout effect of the compound is stronger as the dosage is continuously increased. The compounds of the above Examples have remarkably higher swelling inhibition rate for relieving gout symptoms than the methylprednisolone (53.83%), and with the substituted bis-oxime group, the *in vivo* metabolic cycle is prolonged, so as to have a relatively long-term effect of relieving gout.

### Experiment 2

The compounds prepared in Examples 1-17 have strong anti-inflammatory efficacy.

The anti-inflammatory effects of the compounds prepared in the above Examples were detected using a carrageenan-induced mouse paw swelling model, and the control sample was methylprednisolone:
Carrageenan-induced mouse paw swelling model is a classical acute inflammation model commonly used for evaluating or screening anti-inflammatory effects of drugs. When carrageenan is injected into the paw of mice, a series of reactions similar to human acute inflammation such as local capillary dilatation, increased vascular permeability, exudation and edema can be caused, so the model can be widely used in the evaluation of new drugs.

Reference for experimental method: Yang Huan, Cheng Xiao, Wang Yuehua, Du Guanhua. Effects of esculin on the swelling of toes and inflammation induced by carrageenan in mice [J]. Chinese Journal of New Drugs, 2016(25):2322.

On the day of the experiment, the circumference of right hind paw (cm) of each mouse was measured firstly, and then, after the onset of inflammation, the circumference of right hind paw was measured so that the difference of the circumferences of the paws before and after inflammation was swelling degree, and the swelling inhibition rate of paw (swelling inhibition rate %=(C-T)/C* 100%); C= swelling rate of the control group %, and T= swelling rate of the treatment group) was calculated.
(1) The dosage is positively correlated with the anti-inflammatory effect, and the specific experimental results are as follows: 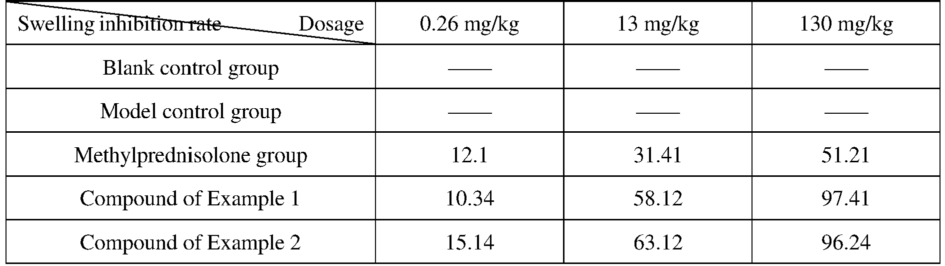 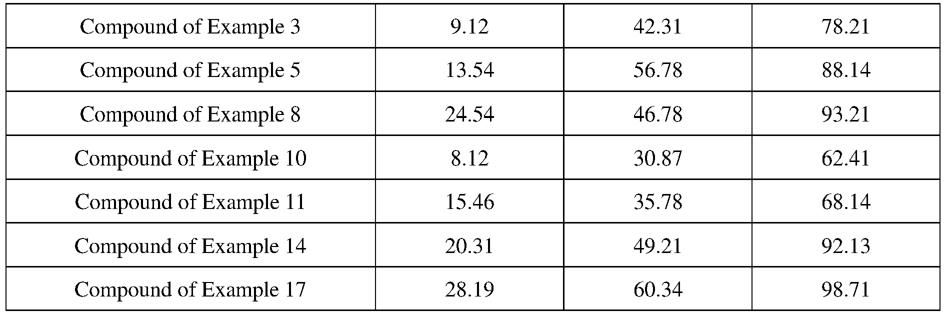
(2) Gavage administration was performed once a day for seven consecutive days. The circumference of the right hind paw was marked and measured before the last administration, and the average value of the two measurements was taken as the standard. 30 min after the last administration, 20 µL/mice of 0.5% carrageenan was injected into the right hind ankle of the mice to induce inflammation, and the circumference of paw was measured 180 min after the onset of inflammation. The results of administering to mice 130 mg/kg methylprednisolone, and the compound of the above Example were as follows:

| | Circumference of the paw before administration (cm) | Circumference of the paw after administration (cm) | Swelling value (cm) | Swelling degree (%) | Swelling inhibition rate (%) |
|---|---|---|---|---|---|
| Blank control group | 1.48 | 1.48 | 0.00 | 0.00 | - |
| Model control group | 1.55 | 1.85 | 0.30 | 19.35 | - |
| Methylprednisolone group | 1.48 | 1.63 | 0.15 | 10.14 | 47.64 |
| Compound of Example 1 | 1.45 | 1.46 | 0.01 | 0.69 | 96.44 |
| Compound of Example 2 | 1.42 | 1.43 | 0.01 | 0.7 | 96.36 |
| Compound of Example 3 | 1.48 | 1.53 | 0.05 | 3.38 | 82.55 |
| Compound of Example 5 | 1.48 | 1.52 | 0.04 | 2.7 | 86.04 |
| Compound of Example 8 | 1.47 | 1.49 | 0.02 | 1.36 | 92.97 |
| Compound of Example 10 | 1.47 | 1.58 | 0.11 | 7.48 | 61.34 |
| Compound of Example 11 | 1.43 | 1.53 | 0.1 | 6.99 | 63.87 |
| Compound of Example 14 | 1.42 | 1.45 | 0.03 | 2.11 | 89.08 |
| Compound of Example 17 | 1.42 | 1.43 | 0.01 | 0.7 | 96.36 |

In this experiment, the ICR mice were consecutively administered different doses of the compounds one week before the plantar injection of 0.5% carrageenan. The test results showed that the swelling inhibition rate (%) of the compounds of the above Examples was in the range of 61.34% to 96.44%, and within the safe dose, the anti-inflammatory of the compound is stronger as the dosage is continuously increased; the compounds prepared in the above Examples have better inhibitory effects on paw swelling than methylprednisolone (47.64%), and with the substituted bis-oxime group, the *in vivo* metabolic cycle is prolonged, so as to have a relatively long-term anti-inflammatory effect.

From the perspective of biochemical indicators, when an inflammatory reaction occurs, the expression level of glucocorticoid receptor α (GRa) protein is inhibited, the expression level of glucocorticoid receptor β (GRb) protein is promoted, the expression level of proinflammatory factor TNF-α protein is significantly increased, and the expression of inflammatory protective factor IL-10 is inhibited. Experiments show that the compounds of the above Examples can promote the expression of the GRa protein, inhibit the expression of the GRb protein, inhibit the increase of TNF-α, and increase the amount of IL-10 until it returns to normal.

### Experiment 3 Acute toxicity test

The experiment was designed according to the "Technical Guideline for Acute Toxicity Test of Chemical Drugs" issued by the State Food and Drug Administration in March 2005. The doses administered were 4 g/kg, 4.91 g/kg, 6.14 g/kg, 7.68 g/kg, 9.6 g/kg, 12 g/kg, and 15 g/kg (the dose could be adjusted according to the actual test results), 12 ICR mice per dose group. If the administration volume is greater than 0.5 ml, it is better to perform the drug administration in two or more times (Note: according to the national standard GB 15193.3-2014, if multiple drug administrations are required, the time interval should be 4 h to 6 h each time, and the patient should be fasted on the day before drug administration). The results of the acute toxicity tests are shown in the following table:

| Mortality (deaths /12) | 4 g/kg | 4.91 g/kg | 6.14 g/kg | 7.68 g/kg | 9.6 g/kg | 12 g/kg | 15 g/kg |
|---|---|---|---|---|---|---|---|
| Methylprednisolone (p.o.) | 0/12 | 0/12 | 0/12 | 1/12 | 3/12 | 5/12 | 7/12 |
| Compound of Example 1 (p.o.) | 0/12 | 0/12 | 0/12 | 0/12 | 0/12 | 0/12 | 0/12 |
| Compound of Example 2 (p.o.) | 0/12 | 0/12 | 0/12 | 0/12 | 1/12 | 4/12 | 6/12 |
| Compound of Example 3 (p.o.) | 0/12 | 0/12 | 0/12 | 0/12 | 0/12 | 0/12 | 1/12 |
| Compound of Example 5 (p.o.) | 0/12 | 0/12 | 0/12 | 0/12 | 1/12 | 2/12 | 4/12 |
| Compound of Example 8 (p.o.) | 0/12 | 0/12 | 0/12 | 0/12 | 0/12 | 0/12 | 1/12 |
| Compound of Example 9 (p.o.) | 0/12 | 0/12 | 0/12 | 0/12 | 0/12 | 0/12 | 2/12 |
| Compound of Example 10 (p.o.) | 0/12 | 0/12 | 1/12 | 1/12 | 3/12 | 4/12 | 5/12 |

| | | | | | | | |
|---|---|---|---|---|---|---|---|
| Compound of Example 11 (p.o.) | 0/12 | 0/12 | 0/12 | 0/12 | 0/12 | 0/12 | 0/12 |
| Compound of Example 14 (p.o.) | 0/12 | 0/12 | 0/12 | 0/12 | 1/12 | 1/12 | 3/12 |
| Compound of Example 17 (p.o.) | 0/12 | 0/12 | 0/12 | 0/12 | 0/12 | 0/12 | 0/12 |

Although the present invention has been described in detail by general description and specific embodiments, some modifications or improvements can be made on the basis of the present invention, which is obvious to a person skilled in the art. Therefore, all such modifications or improvements made without departing from the spirit of the present invention are within the scope of the present invention.

### Industrial applicability

The present invention provides a compound for treating gout, a preparation method therefor and the use thereof. The compound is as represented by formula I. Disclosed in the present invention are a method for preparing the compound and the use of the compound in the preparation of a drug for treating or preventing gout. Further disclosed in the present invention is a composition product containing the compound. The compound provided by the present invention can be used for treating inflammation and gout, and can also be used for treating cancers, and has good economic value and application prospect.

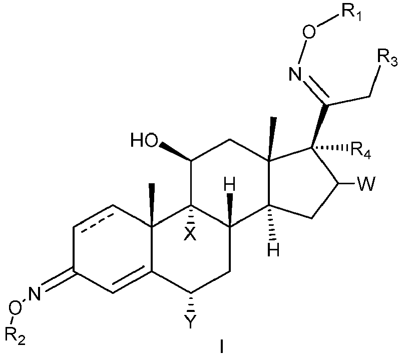

## Claims

1. A compound of formula I, 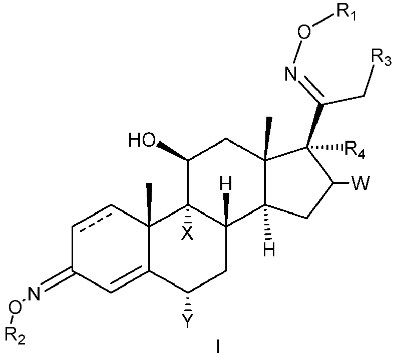 wherein,
R₁ is -H, (C₁-C₄) alkyl or -CH₂-Ar;
R₂ is -H, (C₁-C₄) alkyl or -CH₂-Ar;
R₃ is -H, -OH, halogen, -OC(=O)-(CH₂)n-CH₃ or -OC(=O)-Ar; n is an integer from 0 to 4;
R₄ is -H, -OH, -OC(=O)-(CH₂)m-CH₃ or -OC(=O)-Ar; m is an integer from 0 to 4;
X and Y are independently selected from H, -CH₃ or halogen;
W is H, -CH₃, or 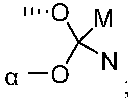 and
M and N are independently selected from H, (C₁-C₄) alkyl, -Ar (aryl), heteroaryl or benzyl;
or a tautomer, a mesomer, a racemate, an enantiomer, a diastereomer, a prodrug or a mixture thereof; or pharmaceutically acceptable salts or solvates or hydrates or polymorphs of the compound of formula I or its tautomer, mesomer, racemate, enantiomer, diastereomer, prodrug or a mixture thereof.

2. The compound of formula I, or a tautomer, a mesomer, a racemate, an enantiomer, a diastereomer, a prodrug or a mixture thereof; or pharmaceutically acceptable salts or solvates or hydrates or polymorphs of the compound of formula I or its tautomer, mesomer, racemate, enantiomer, diastereomer, prodrug or a mixture thereof according to claim 1, wherein,
R₁ is -H or -CH₂CH₃; and/or,
R₂ is -H or -CH₂CH₃; and/or,
R₃ is -H, -OH, -Cl or -OAc, 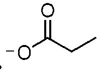 or -CH₂CH₃; and/or,
R₄ is -H, -OH, 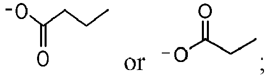 and/or,
X is -H, -F or -Cl; and/or,
Y is -H, -CH₃, -F, or -Cl; and/or,
W is -H, α-CH₃, β-CH₃, α-OH, β-OH,

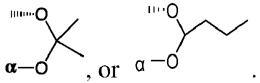

3. The compound of formula I or a tautomer, a mesomer, a racemate, an enantiomer, a diastereomer, a prodrug or a mixture thereof; or pharmaceutically acceptable salts or solvates or hydrates or polymorphs of the compound of formula I or its tautomer, mesomer, racemate, enantiomer, diastereomer, prodrug or a mixture thereof according to claim 1, wherein,
R₁ is -H or -CH₂CH₃; and/or,
R₂ is -H or -CH₂CH₃; and/or,
R₃ is -OH or -OAc; and/or,
R₄ is -OH or -OAc; and/or,
X is -H; and/or,
Y is -H or -CH₃; and/or,
W is -H.

4. A compound of formula II: 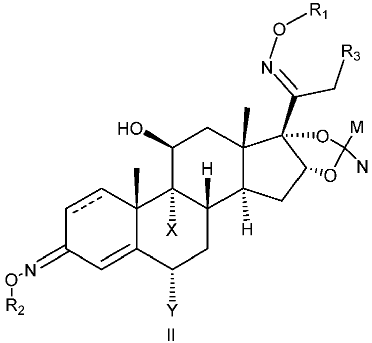
wherein, R₁, R₂, R₃, X, Y, M and N have the same definitions as any one of claims 1 to 3; and
preferably, M and N are independently selected from H, (C₁-C₄) alkyl, -Ar, heteroaryl or benzyl;
or a tautomer, a mesomer, a racemate, an enantiomer, a diastereomer, a prodrug or a mixture thereof; or pharmaceutically acceptable salts or solvates or hydrates or polymorphs of the compound of formula II or its tautomer, mesomer, racemate, enantiomer, diastereomer, prodrug or a mixture thereof.

5. Compounds of the following structures: 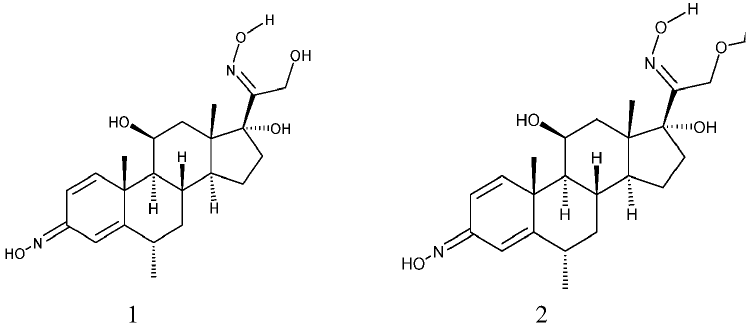 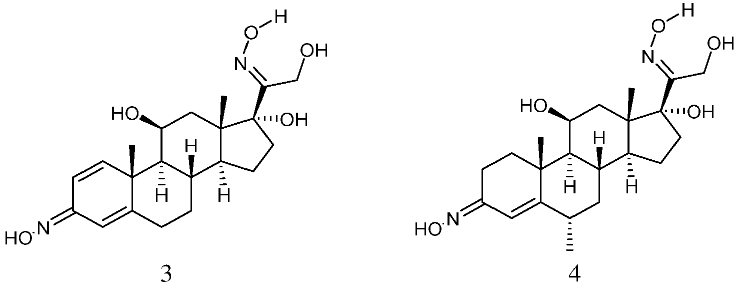 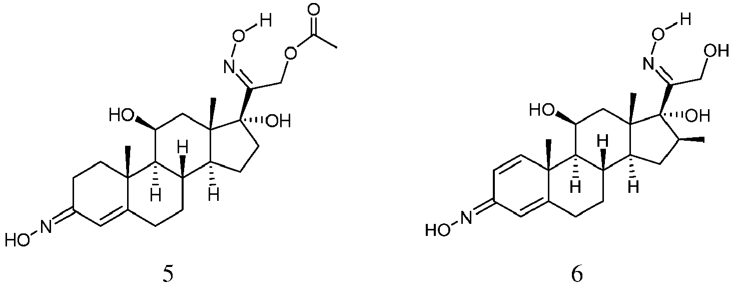 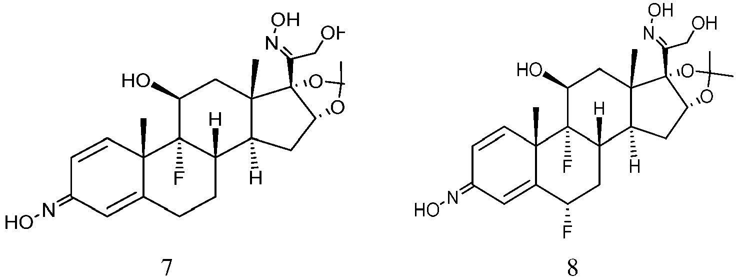 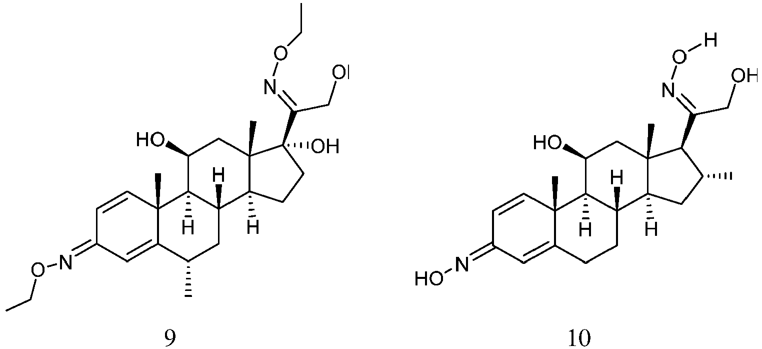 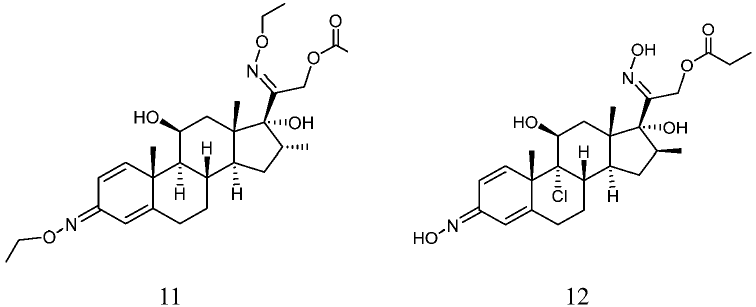 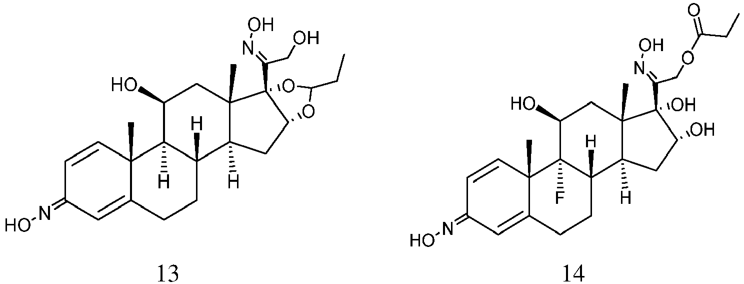 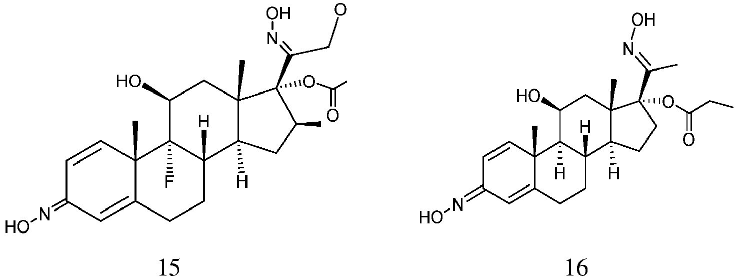 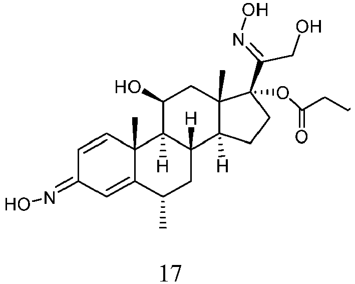 or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof.

6. A method for preparing the compound of formula I, or a tautomer, a mesomer, a racemate, an enantiomer, a diastereomer, a prodrug or a mixture thereof; or pharmaceutically acceptable salts or solvates or hydrates or polymorphs of the compound of formula I or its tautomer, mesomer, racemate, enantiomer, diastereomer, prodrug or a mixture thereof according to claim 1, comprising the following synthetic routes: 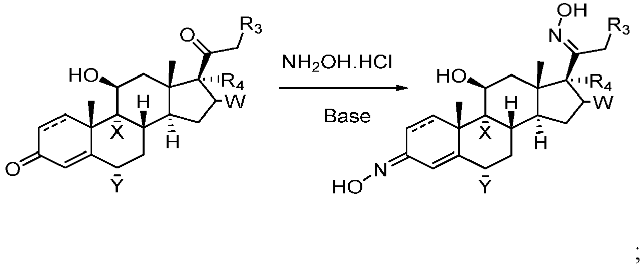 or 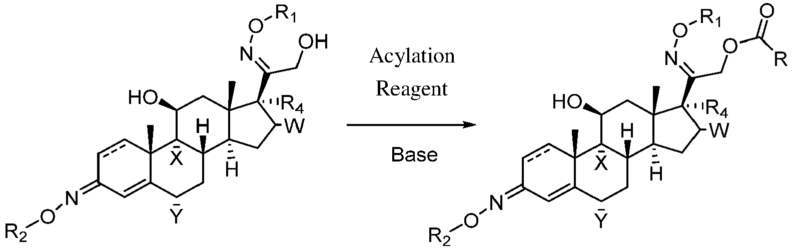 ; or 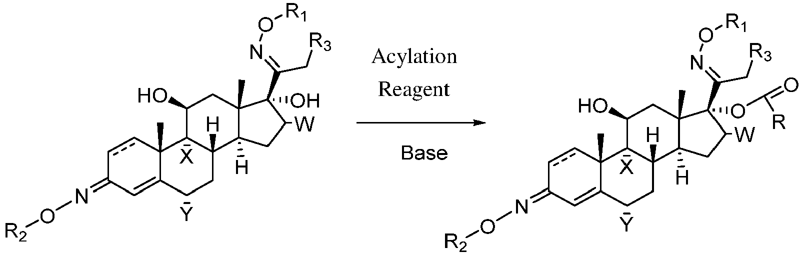 or 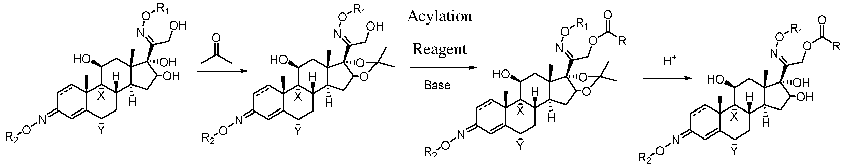 or 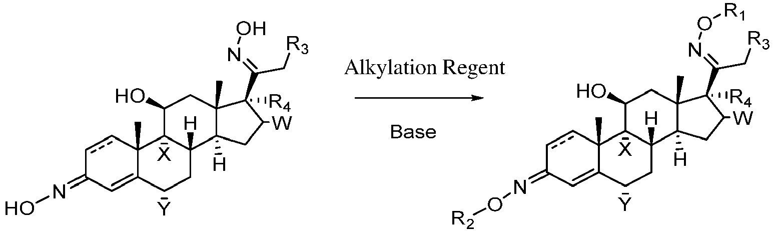
wherein R₁, R₂, R₃ R₄, X, Y, W, M and N have the same definitions as any one of claims 1 to 3;
R is a linear saturated alkyl, preferably a linear saturated (C₁-C₁₂) alkyl, and more preferably a linear saturated (C₁-C₄) alkyl such as methyl, ethyl, n-propyl or n-butyl.

7. A method for preparing the compound of formula II, or a tautomer, a mesomer, a racemate, an enantiomer, a diastereomer, a prodrug or a mixture thereof; or pharmaceutically acceptable salts or solvates or hydrates or polymorphs of the compound of formula II or its tautomer, mesomer, racemate, enantiomer, diastereomer, prodrug or a mixture thereof according to claim 4, comprising the following synthetic routes: 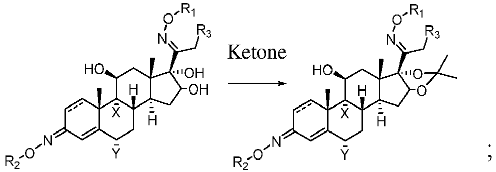 or 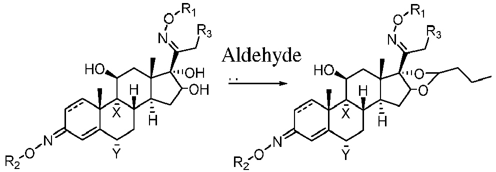
wherein R₁, R₂, R₃, X, Y, W, M and N have the same definitions as any one of claims 1 to 3; and
preferably, M and N are independently selected from H, (C₁-C₄) alkyl, -Ar, heteroaryl or benzyl.

8. Use of the compound or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof in the preparation of a drug for treating or preventing gout, or in the preparation of a drug for treating or preventing inflammation; or in the preparation of a drug for treating or preventing cancer (or tumor); preferably, the drug further comprises a pharmaceutically acceptable auxiliary material;
wherein, the gout is preferably a gout in the acute stage;
the inflammation is preferably an inflammation associated with gout in the acute stage, and more preferably an inflammation associated with regulating any one or more of the expressions of glucocorticoid receptor GRa, glucocorticoid receptor GRb, proinflammatory factor TNF-α, and inflammatory protective factor IL-10 in the inflammatory response pathway;
the cancer is preferably any one or more of human lung adenocarcinoma, human acute lymphoblastic leukemia, human B cell lymphoma, human breast carcinoma, human malignant glioblastoma, human live cancer, human cervical cancer, human non-small cell lung cancer, human osteosarcoma, human colon cancer, human osteoblastic sarcoma, and human Burkitt's lymphoma;
further preferably, the compound or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof in the use is as the sole active ingredient or in combination with other biologically active substances.

9. A combination product for treating or preventing gout or inflammation or cancer (or tumor), wherein the combination product comprises one or more of the compound or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof according to any one of claims 1 to 5; preferably further comprises a pharmaceutically acceptable auxiliary material; and/or the combination product is a kit.

10. A method for treating or preventing gout or inflammation or cancer, comprising: administering to a subject or patient in need thereof, orally or non-orally, an effective amount of the compound or a tautomer, a mesomer, a racemate, an enantiomer, a diastereomer, a prodrug or a mixture thereof; or pharmaceutically acceptable salts or solvates or hydrates or polymorphs of the compound or its tautomer, mesomer, racemate, enantiomer, diastereomer, prodrug or a mixture thereof according to any one of claims 1 to 5, or the combination product according to claim 9;
wherein the gout is preferably a gout in the acute stage;
the inflammation is preferably an inflammation associated with regulating any one or more of the expressions of glucocorticoid receptor GRa, glucocorticoid receptor GRb, proinflammatory factor TNF-α, and inflammatory protective factor IL-10 in the inflammatory response pathway;
the cancer is preferably any one or more of human lung adenocarcinoma, human acute lymphoblastic leukemia, human B cell lymphoma, human breast carcinoma, human malignant glioblastoma, human live cancer, human cervical cancer, human non-small cell lung cancer, human osteosarcoma, human colon cancer, human osteoblastic sarcoma, and human Burkitt's lymphoma.
